# EUROPEAN PATENT SPECIFICATION

(11) **EP 2 271 746 B1**
(45) Date of publication and mention of the grant of the patent: **15.04.2015**
(21) Application number: 09710897.1
(22) Date of filing: 10.02.2009
(51) Int. Cl.: C12N 5/16, C12N 5/074

(54) **METHOD FOR REPROGRAMMING DIFFERENTIATED CELLS**
VERFAHREN ZUR UMPROGRAMMIERUNG AUSDIFFERENZIERTER ZELLEN
PROCÉDÉ POUR LA REPROGRAMMATION DE CELLULES DIFFÉRENCIÉES

(30) Priority: 13.02.2008 EP 08101576
(43) Date of publication of application: 12.01.2011
(73) Proprietor: Cosma, Maria Pia, Salerno (IT); Viñas, Frederic Lluis, Mollet del Vallés, Barcelona (ES)
(72) Inventor: Cosma, Maria Pia, Salerno (IT); Viñas, Frederic Lluis, Mollet del Vallés, Barcelona (ES)
(74) Representative: Capasso, Olga
(86) International application number: PCT/EP2009/051512
(87) International publication number: WO 2009/101084

(56) References cited:
- SILVA JOSE ET AL: "Nanog promotes transfer of pluripotency after cell fusion" NATURE, NATURE PUBLISHING GROUP, LONDON, vol. 441, no. 7096, 1 June 2006 (2006-06-01), pages 997-1001, XP002425046 ISSN: 0028-0836
- ALBERIO R ET AL: "Reprogramming somatic cells into stem cells" REPRODUCTION 200611 GB, vol. 132, no. 5, November 2006 (2006-11), pages 709-720, XP002481665 ISSN: 1470-1626
- AMBROSI DOMINIC J ET AL: "Genome-wide reprogramming in hybrids of somatic cells and embryonic stem cells" STEM CELLS (MIAMISBURG), vol. 25, no. 5, 2007, pages 1104-1113, XP002481666 ISSN: 1066-5099
- COWAN CHAD A ET AL: "Nuclear reprogramming of somatic cells after fusion with human embryonic stem cells" SCIENCE, WASHINGTON, DC, vol. 309, no. 5739, 26 August 2005 (2005-08-26), pages 1369-1373, XP002425303 ISSN: 0036-8075
- MARSON ALEXANDER ET AL: "Wnt signaling promotes reprogramming of somatic cells to pluripotency" CELL STEM CELL, vol. 3, no. 2, August 2008 (2008-08), pages 132-135, XP002523704 ISSN: 1934-5909
- LLUIS FREDERIC ET AL: "Periodic Activation of Wnt/beta-Catenin Signaling Enhances Somatic Cell Reprogramming Mediated by Cell Fusion" CELL STEM CELL, vol. 3, no. 5, November 2008 (2008-11), pages 493-507, XP002523705 ISSN: 1934-5909
- TAKAHASHI K ET AL: "Induction of pluripotent stem cells from mouse embryonic and adult fibroblast cultures by defined factors" CELL, CELL PRESS, CAMBRIDGE, NA, US, vol. 126, no. 4, 25 August 2006 (2006-08-25), pages 663-676, XP003013968 ISSN: 0092-8674
- CHUNG YOUNG ET AL: "Human embryonic stem cell lines generated without embryo destruction", CELL STEM CELL, CELL PRESS, US, vol. 2, no. 2, 7 February 2008 (2008-02-07), pages 113-117, XP002604696, ISSN: 1934-5909, DOI: 10.1016/J.STEM.2007.12.013
- KANATSU-SHINOHARA M ET AL: "The germ of pluripotency", NATURE BIOTECHNOLOGY, NATURE PUBLISHING GROUP, NEW YORK, NY, US, vol. 24, no. 6, 1 June 2006 (2006-06-01), pages 663-664, XP002429562, ISSN: 1087-0156, DOI: 10.1038/NBT0606-663
- M Z Ratajczak ET AL: "A hypothesis for an embryonic origin of pluripotent Oct-4+ stem cells in adult bone marrow and other tissues", Leukemia, 8 March 2007 (2007-03-08), XP055082039, ISSN: 0887-6924, DOI: 10.1038/sj.leu.2404630
- ABHISHEK SOHNI ET AL: "Multipotent adult progenitor cells", BEST PRACTICE & RESEARCH CLINICAL HAEMATOLOGY, vol. 24, no. 1 , pages 3-11, XP028177075, ISSN: 1521-6926, DOI: 10.1016/J.BEHA.2011.01.006 [retrieved on 2011-01-30]
- Daniela Sanges ET AL: "Wnt/[beta]-Catenin Signaling Triggers Neuron Reprogramming and Regeneration in the Mouse Retina", Cell Reports, vol. 4, no. 2, 1 July 2013 (2013-07-01), pages 271-286, XP055117186, ISSN: 2211-1247, DOI: 10.1016/j.celrep.2013.06.015

## Description

### Field of the invention

The present invention relates to a method for reprogramming a differentiated cell to an undifferentiated cell by means of a Wnt/β-catenin activator or of a GSK-3 inhibitor.

### Background of the invention

The Wnt/β-catenin signalling pathway regulates a variety of cellular processes during the development of vertebrates and invertebrates, including cell proliferation and differentiation, cell fate, and organogenesis (Karner et al., 2006; Reya and Clevers, 2005). In addition, the Wnt/β-catenin pathway controls tissue homeostasis and regeneration in response to damage in Zebra fish, *Xenopus,* planarians, and even adult mammals (Gurley et al., 2008; Ito et al., 2007; Osakada et al., 2007; Petersen and Reddien, 2008; Stoick-Cooper et al., 2007; Yokoyama et al., 2007).

β-catenin links cadherins to the cytoskeleton in cell-cell adhesion and acts as the intracellular signalling molecule of the Wnt pathway. The binding of Wnt ligands to Frizzled and LRP5/6 receptors results in inactivation of a multiprotein destruction complex, which is composed of glycogen synthase kinase-3 (GSK-3), Axin and adenomatous polyposis coli (APC). This inactivation leads to a decrease in β-catenin phosphorylation, which then allows β-catenin to escape ubiquitin-proteasome-mediated degradation (Willert and Jones, 2006). As a consequence, non-degraded β-catenin accumulates in the cytoplasm and translocates into the nucleus, leading to transcriptional activation of a plethora of target genes that control a variety of cellular and tissue processes (Hoppler and Kavanagh, 2007). In the absence of Wnt activation, β-catenin is phosphorylated by the destruction complex and degraded by the ubiquitin-proteasome system. Activation of Wnt/β-catenin signalling induces the expression of multiple antagonists that act in a coordinated manner to shut down the pathway at many levels simultaneously. Extracellularly, the Wif-1 and Dkk proteins inhibit Wnt signalling by interacting with the Wnt protein and LRP5/6 receptors, respectively (Kawano and Kypta, 2003). Intracellularly, both Nkd-1 and Axin2 enhance β-catenin degradation through an increase in the formation of the destruction complex (Behrens, 2005; Wharton et al., 2001). The Wnt proteins form a large family of isoforms (from Wnt1 to Wnt10b, Wnt11 and Wnt16) (Kikuchi et al., 2007). Among these, Wnt3a has been shown to enhance self-renewal and to maintain totipotency of embryonic stem (ES) cells and haematopoietic stem cells (HSCs) (Anton et al., 2007; Reya et al., 2003) through the accumulation of β-catenin in the cell nucleus. Likewise, both inactivation of the *GSK-3α* and *GSK-3*β homologues and mutations in *APC* lead to β-catenin stabilization and severely compromise the ability of ES cells to differentiate and to maintain their sternness (Doble et al., 2007; Kielman et al., 2002; Sato et al., 2004).

Reprogramming can allow de-differentiation of differentiated cells to pluripotency and can take place via different mechanisms. Somatic nuclear transfer has allowed the cloning of different animals through the reprogramming of a differentiated nucleus (Vajta, 2007). Recent findings have shown that over-expression of four specific ES-cell transcription factors encoding genes in mouse and human somatic cells can induce them to become pluripotent (Aoi et al., 2008; Brambrink et al., 2008; Hanna et al., 2008; Kim et al., 2008; Lowry et al., 2008; Maherali et al., 2007; Park et al., 2008; Takahashi et al., 2007; Takahashi and Yamanaka, 2006; Yu et al., 2007). Somatic mouse and human cells can also be reprogrammed by fusion with ES cells, and Nanog, a factor that maintains ES cells in their undifferentiated state, augments the pluripotency of the hybrids (Cowan et al., 2005; Silva et al., 2006; Tada et al., 1997; Tada et al., 2001). In ES-cell-thymocyte hybrids, reactivation of the inactive X chromosome, erasure of DNA methylation associated with imprinted genes, and reactivation of the Oct4-GFP transgene have all been seen. Moreover, when these hybrid cells were introduced into host diploid blastocysts, they contributed to the three germ layers of the chimeric embryos (Tada et al., 1997; Tada et al., 2001). Similar findings have been reported after the fusion of human fibroblasts with human ES cells. The tetraploid hybrids obtained showed the phenotype and growth rate of the human ES cells. The somatic genome was reprogrammed, as demonstrated by human-ES-cell-like genome-wide transcriptional analysis (Cowan et al., 2005).

Patent application WO 2007/115216 provides methods for therapeutically reprogramming human adult stem cells into pluripotent cells. Such methods consist in isolating a human adult stem cell and placing it in contact with a medium comprising stimulatory factors which induce the development of the stem cell into a therapeutically reprogrammed cell. The medium comprises PM-10^{™}. Such method has been also proposed in the patent application WO 2005/123123 wherein the stimulatory factor is a chemical selected from the group consisting of 5-aza-2'-deoxycytidine, histone de-acetylase inhibitor, n-butyric acid and trichostatin.

Patent application WO 2007/054720 discloses methods for reprogramming and genetically modifying cells. In this application a pluripotent genome is obtained from a differentiated genome by fusing a pluripotent cell with a differentiated cell in the presence of Nanog or a MEK inhibitor.

Patent application WO 2007/019398 provides improved methods for reprogramming an animal differentiated somatic cell to an undifferentiated stem cell. Such methods comprise (a) injecting one or more animal differentiated somatic cells into an oocyte, (i) remodelling the nuclear envelope of the nucleus of the somatic cell, and (ii) reprogramming the chromatin of the somatic cell and (b) transferring or fusing the resulting remodelled nucleus obtained in (a) into the enucleated cytoplasm of an undifferentiated embryonic cell to form an undifferentiated stem cell.

Patent application WO 2007/016245 relates to methods of reprogramming adult stem cells or stem cells obtained from umbilical or placental cord blood or from the placenta or umbilical cord tissue itself or the amniotic fluid or amnion. The reprogrammed stem cells are obtained by exposing adult pluripotent stem or progenitor cells or pluripotent stem or progenitor cells obtained from placental or umbilical cord tissue or placental or umbilical cord blood or amniotic fluid or amnion to an effective amount of embryonic stem cell medium or to a denucleation procedure to remove nuclear DNA from the stem cell followed by introduction of nuclear material from a cell of a patient to be treated. The embryonic stem cell medium is a minimum essential medium comprising at least one growth factor, glucose, nonessential amino acids, insulin and transferrin. The growth factor is fibroblast growth factor, transforming growth factor beta or mixtures thereof and said medium further comprises at least one additional component selected from the group consisting gamma amino butyric acid, pipecholic acid, lithium and mixtures thereof.

Patent application WO 2005/033297 discloses compositions, methods and kits for non-nuclear transfer reprogramming an adult differentiated cell obtained from an adult tissue into an ES-like cell. The method of producing a reprogrammed embryonic stem-cell like cell comprises contacting a differentiated adult cell with a Mycobacterium leprae bacterium, or a component thereof selected from the group consisting of a PGL-1, a whole cell wall fraction, a cell wall protein, a cell wall lipid, a cell wall carbohydrate, a protein released from a viable bacterium, and a protein secreted from a viable bacterium.

Patent application WO 2005/068615 provides a method of culturing a pluripotent stem cell while inhibiting differentiation of the pluripotent stem cell by suppression of Wnt/β-catenin signaling. The Wnt/β-catenin signalling suppressor is sFRP, collagen XVIII, endostatin, carboxypeptidase Z, receptor tyrosine kinase, transmembrane enzyme Corin, WIF-1, Cerebus, Dickkopf-1, or a combination thereof.

Patent application WO 2007/016485 discloses the use of a GSK-3 inhibitor to maintain potency of cultured cells. This document is directed to the culture of non-embryonic cells, that can differentiate into cell types of more than one embryonic lineage, in culture under conditions that maintain differentiation capacity during expansion; more particularly, culturing non-embryonic cells in the presence of at least one GKS-3 inhibitor, such as BIO. Thus, several strategies have been proposed to reprogram differentiated cells such as somatic cell nuclear transplantation, treatment with extracts of pluripotent cells or stable expression of defined factors. However such strategies present several drawbacks including limited application due to availability of oocytes and low cloning efficiency, reprogrammed cell regaining only some of the properties of the pluripotent cells or elicitation of side effects. In addition, reprogramming via treatment with extracts of pluripotent cells leads to very transient, if any, reprogramming of the differentiated cells. Furthermore, the reprogramming via the stable expression of defined factors implies the use of ES-specific factors (Oct4, klf4, sox2, lin28) or one oncogene (myc) which may lead to undesired side effects.

Therefore there is the need to provide a method for reprogramming a somatic cell that display high efficiency of reprogramming and that is more physiological.

### Description of the invention

The present invention relates to a method for reprogramming a differentiated cell to an undifferentiated stem cell by means of the use of a Wnt/β-catenin activator or of a GSK-3 inhibitor.

Since Wnt/β-catenin signalling controls ES self-renewal and the maintenance of sternness (Sato et al., 2004), and regulates the expression of ES-cell genes (Cole et al., 2008), authors hypothesized that the Wnt/β-catenin signalling pathway can also control the reprogramming of somatic cells to pluripotency.

As a matter of facts, the authors show that transient activation of Wnt/β-catenin signaling can trigger reprogramming of three different types of somatic cells. Large numbers of reprogrammed colonies can be generated after fusion of ES cells with NS cells, mouse embryonic fibroblasts or thymocytes, by culturing the hybrids transiently with Wnt3a or a glycogen synthase kinase-3 (GSK-3) inhibitor such as 6-bromoindirubin-3'-oxime (BIO) and CHIR99021. The isolated reprogrammed clones express ES-cell-specific genes, lose somatic differentiation markers, become unmethylated on *Oct4* and *Nanog* CpG islands, and can differentiate into cardiomyoctes *in vitro* and generate teratomas *in vivo.* The isolated reprogrammed clones are pluripotent, since they are able to differentiate *in vitro* and *in vivo.* Then the authors identify Wnt/β-catenin as the first pathway that is not constitutively active in ES cells that can kick-off cell reprogramming. This pathway represents a toggle switch for initiation of the cascade of events that leads to reprogramming of somatic cells in living organisms.

Therefore it is an object of the present invention a method for reprogramming a differentiated cell to an undifferentiated stem cell comprising the step of fusing said differentiated cell to an isolated pluripotent cell, wherein said isolated pluripotent cell is treated in vitro with a suitable amount of a Wnt/β-catenin pathway activator being at least one Wnt protein isoform or a glycogen synthase kinase-3 inhibitor.

Preferably, the isolated pluripotent cell is treated in vitro before or after the step of fusing said differentiated cell to said pluripotent cell.

Preferably the isolated pluripotent cell is an adult stem cell. Such cell may be obtained by different methods known in the art, also not implying destruction of human embryos. More preferably the isolated pluripotent cell is an embryonic stem cell. Such cell may be obtained by different methods known in the art, also not implying destruction of human embryos.

In a particular embodiment the differentiated cell is a somatic cell. Preferably the differentiated cell is a neural stem cell. Alternatively the differentiated cell is an embryonic fibroblast. Alternatively the differentiated cell is a thymocyte.

Still preferably the isolated pluripotent cell is a mouse or a human cell and the differentiated cell is a mouse or a human cell.

In an embodiment the Wnt/β-catenin pathway activator is at least one Wnt protein isoform. Preferably the Wnt protein isoform is selected from the group of: Wnt1, Wnt2, Wnt2b/13, Wnt3, Wnt3a, Wnt4, Wnt5a, Wnt5b, Wnt6, Wnt7a, Wnt8a, Wnt8b, Wnt9a, Wnt9b, Wnt10a, Wnt10b, Wnt11 or Wnt16. Examples of Wnt protein isoforms are the following or orthologues thereof (Swiss-prot references):
Homo sapiens:Wnt1: P04628; Wnt2: P09544; wnt2b/13: Q93097; wnt3: P56703; wnt3a: P56704; wnt4: P56705; wnt5a: P41221; wnt5b: Q9H1J7; wnt6: Q9Y6F9; wnt8a: Q9H1J5; wnt9a: 014904; wnt9b: 014905; wnt10a: Q9GZT5; wnt10b: 000744; wnt11: 096014; wnt16: Q9UBV4.
Mus musculus: Wnt1: P04426; wnt2: P21552.1; wnt2b/13: 070283.2; wnt3: P17553; wnt3a: P27467; wnt4: P22724; wnt5a: P22725; wnt5b: P22726; wnt6: P22727.1; wnt8a: Q64527; wnt9a: Q8R5M2; wnt9b: 035468.2; wnt10a: P70701; wnt10b: P48614; wnt11: P48615; wnt16: Q9QYS1.1.

In an alternative embodiment the Wnt/beta-catenin pathway activator is a glycogen synthase kinase-3 (GSK3) inhibitor. Preferably, the GSK3 inhibitor is BIO or CHIR99021 or an analogue thereof (Mus musculus: Q9WV60; Homo sapiens: P49841).

In a preferred embodiment the method of the invention further comprises the step of overexpressing Nanog protein in the isolated pluripotent cell, or in the fused cell. Examples of Nanog proteins are the following or orthologues thereof: Mus musculus: Q80Z64; Homo sapiens: Q9H9S0.

Preferably the overexpression of Nanog protein is obtained by genetically modifying the isolated pluripotent cell or the fused cell or by transducing the pluripotent cell or the fused cell with an appropriated vector expressing the Nanog protein.

In the present invention a differentiated cell means a cell specialized for a specific function (such as a heart, liver, or muscle cell) that cannot generate other types of cells. An undifferentiated stem cell is a cell not specialized for a specific function that retains the potential to give rise to specialized cells. A pluripotent cell is a primordial cell that can differentiate into a sub-group of specialized types of cells. An adult stem cell is an undifferentiated cell (called also somatic stem cell) that is present in adult or foetal or new born tissues and is able to differentiate into specialized cells of the tissue from which it originated. An embryonic stem cell is an undifferentiated cell that is present in embryos and is able to differentiate into any type of specialized cell. Such embryonic stem cell, though present in embryos, may also be obtained without embryo destructions. A precursor cell is a cell that detains a precocious state of differentiation and is already committed to differentiate in cell types of a specific lineage. A somatic cell is any cell of a multicellular organism that will not contribute to gamete cells.

The present invention shall be disclosed in detail in the following description also by means of non limiting examples referring to the following figures.

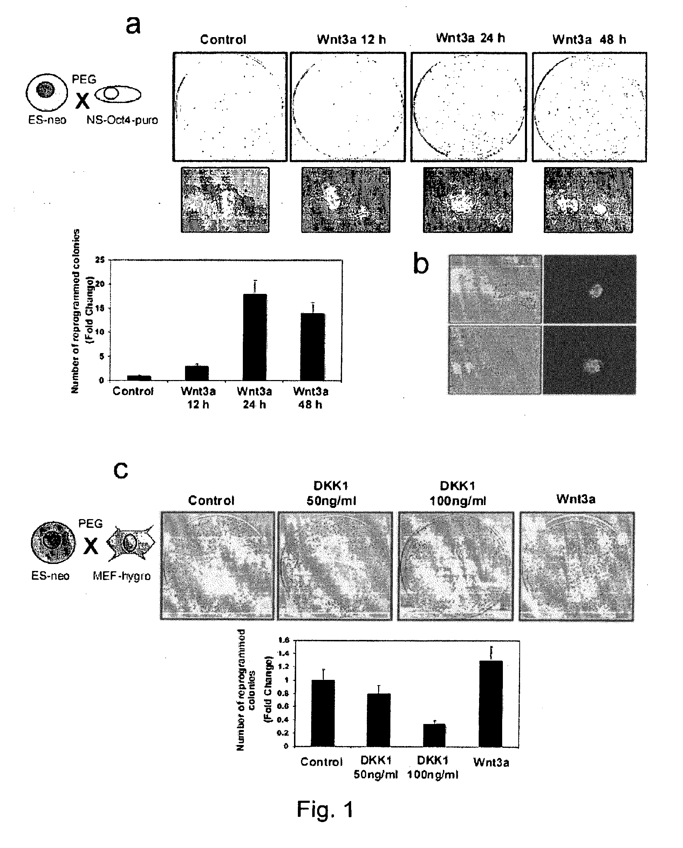

**Figure 1****. Activation of the Wnt pathway by Wnt3a enhances reprogramming mediated by cell fusion. (A)** Representative plates and quantification (as fold-increases in the number of colonies) of hybrid colonies from PEG fusions of ES-neo cells with NS-Oct4-puro cells treated with Wnt3a, as indicated. Colonies were stained for expression of AP and counted (mean ±s.e.m.; n = 3). **(B)** Fluorescence images of some reprogrammed hybrid colonies following 24 h Wnt3a treatment (10x magnification). (**C**) Quantification of hybrid colonies from PEG fusions of ES-neo cells and MEF-hygro cells untreated and treated with Dkk1 and Wnt3a, as indicated (mean ±s.e.m.; n = 3).

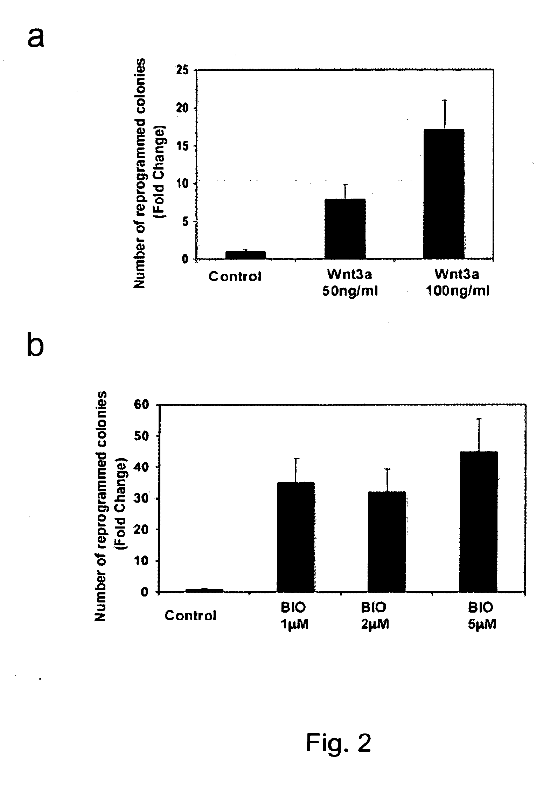

**Figure 2****. Optimization of Wnt3a and BIO concentration for cell reprogramming.** ES-neo x NS-Oct4-puro fusions treated for 24 h with the indicated Wnt3a or BIO concentrations. After fusion and puromycin selection, the colonies were stained for expression of alkaline phosphatase (AP) and counted. The fold-increases in the numbers of colonies are shown (mean ±s.e.m.; n = 3).

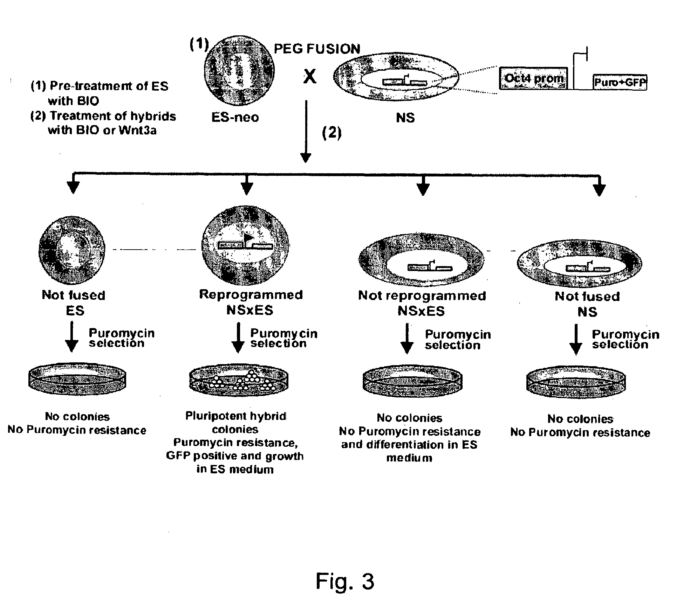

**Figure 3****. Schematic overview of the experimental system.** ES-neo cells expressing pluripotent genes were fused with NS-Oct4-puro cells expressing neural specific genes. To test the role of the Wnt/β-catenin pathway in reprogramming by cell fusion, the cells were treated in two ways: (1) ES-neo cells were treated with BIO for different times before being fused with non-treated NS-Oct4-puro cells; or (2) the cells were first fused, and then treated with Wnt3a or BIO for different times. Only following the full reprogramming of the NS-Oct4-puro cell epigenome will colonies of viable primary hybrids of pluripotent cells with an ES cell phenotype form, because: i) the Oct4 promoter will be active in the NS-cell genome and will express the puromycin resistance gene and GFP; and ii) the hybrid cells will proliferate in ES-cell medium (which contains foetal bovine serum and LIF, two signals that promote differentiation of NS cells).

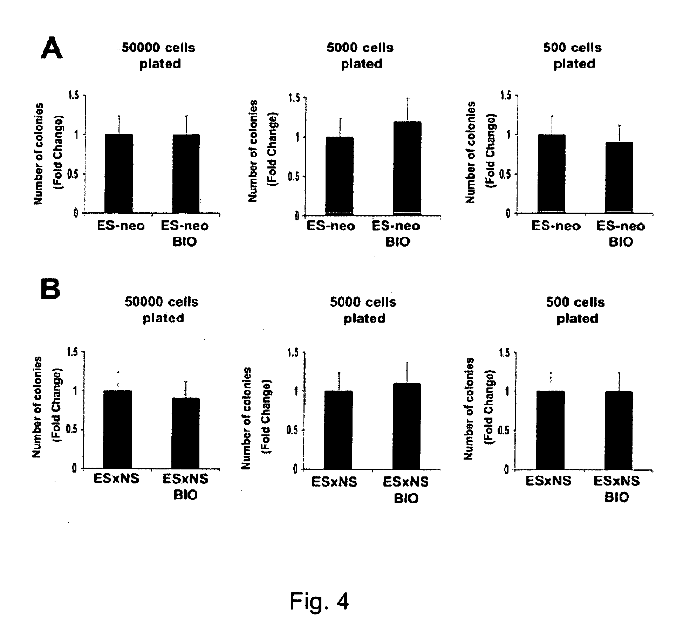

**Figure 4****. BIO treatment of ES cells and ES x NS cells does not enhance cell clonogenicity and viability.** ES-neo cells **(A)** and ES x NS reprogrammed hybrids **(B)** (treated or not for 24 h with BIO) were plated at different densities. After 10 days, the plates were stained for AP and counted. The fold-changes in colonies are shown in the graphs (mean ±s.e.m.; n = 3).

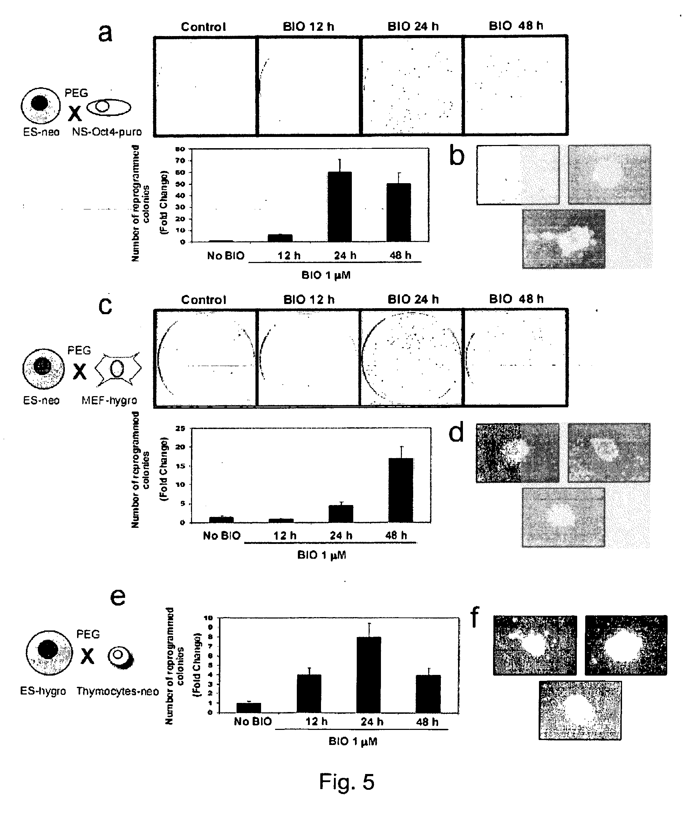

**Figure 5****. Activation of the Wnt/β-catenin pathway by BIO enhances the number of reprogrammed hybrid colonies in three different cell-type fusions. (A)** Quantification (as fold-increases in the number of colonies) of hybrid colonies from PEG fusions of ES-neo cells with NS-Oct4-puro cells treated with BIO, as indicated. (**A, C**) Representative plates and **(A, C, E)** fold-increases in the number of colonies of hybrids clones from the indicated fusions treated with BIO, as indicated, stained for AP expression and counted. The fold-change increases in the reprogrammed colonies are also shown in the graphs (mean ±s.e.m.; n = 3). **(B, D, F)** Light microscopy images of some reprogrammed hybrid colonies following 24 h BIO treatment (10x magnification).

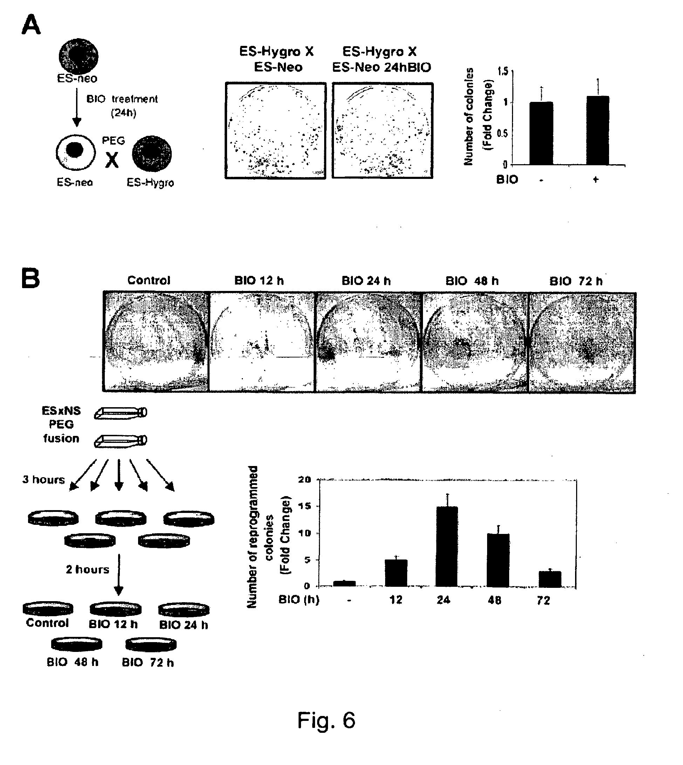

**Figure 6****. BIO treatment of ES cells does not enhance PEG-mediated fusion.** Plates containing fused hybrid colonies between ES-neo cells (treated or not for 24 h with BIO) and non-treated ES-hygro cells. The clones were stained for the expression of AP and counted. The fold-change of colonies are shown in the graphs (mean ±s.e.m.; n = 3). **(B)** Quantification (as fold-increases in the number of colonies) of hybrid colonies from PEG fusions of ES-neo cells with NS-Oct4-puro cells. Hybrids from two fusion experiments (2,000,000 cells/flask) were allowed to recover for 3 h after PEG fusion. They were then pooled and divided in 5 plates (650,000 cells/plate) in such a way that the cells were not touching each other. Two hours after the plating, the cells were treated with BIO, as indicated. The fold-change increases in the reprogrammed colonies are shown in the graphs (mean ±s.e.m.; n = 3).

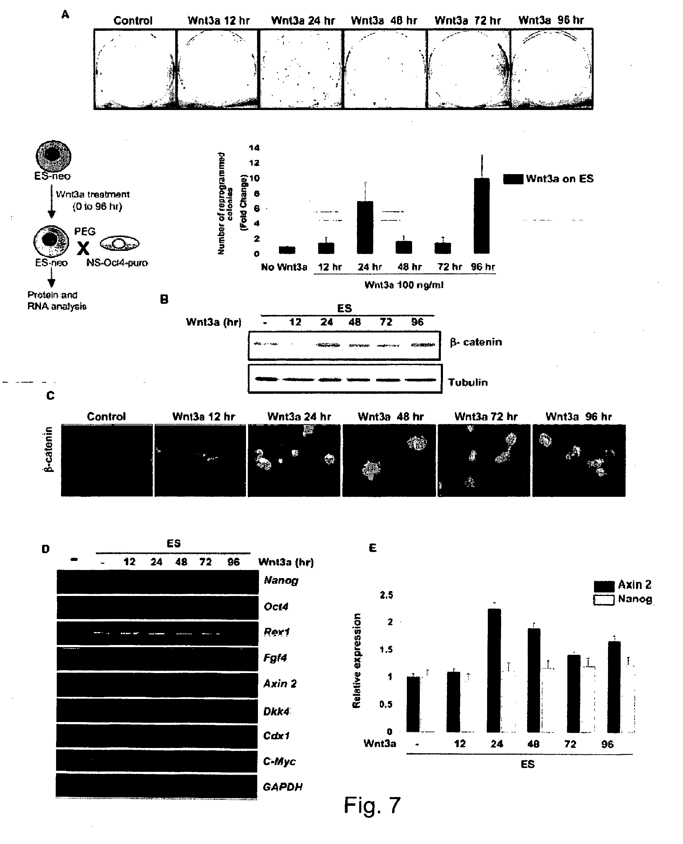

**Figure 7****. Cyclic Wnt/β-catenin signalling pathway activation via Wnt3a enables ES cells to reprogramme NS cells without activation of stem-cell genes. (A)** Representative plates and quantification (as fold-increases in the number of colonies) of hybrid colonies from PEG fusions between Wnt3a-pretreated ES-Neo cells and non-treated NS-Oct4-puro cells, as indicated. Colonies were stained for the expression of AP and counted (mean ±s.e.m.; n = 3). **(B)** Western blotting of protein extracts from ES cells from the Wnt3a-pretreatments in (A), as indicated. **(C)** Immunofluorescence for the visualization of β-catenin localization in ES cells from the Wnt3a-pretreatments in (A), as indicated. **(D)** Semi-quantitative RT-PCR analysis of ES-cell-specific genes (*Nanog, Oct4, Rex1* and *Fgf4*) and β-catenin targets (*Axin2, Cdx1* and *c-Myc*) in total mRNA purified from ES cells from the Wnt3a-pretreatments in (A), as indicated. **(E)** Quantitative RT-PCR analysis of *Axin2* and *Nanog* transcript levels in ES cells from the Wnt3a-pretreatments in (A), as indicated (mean ±s.e.m.; n = 3).

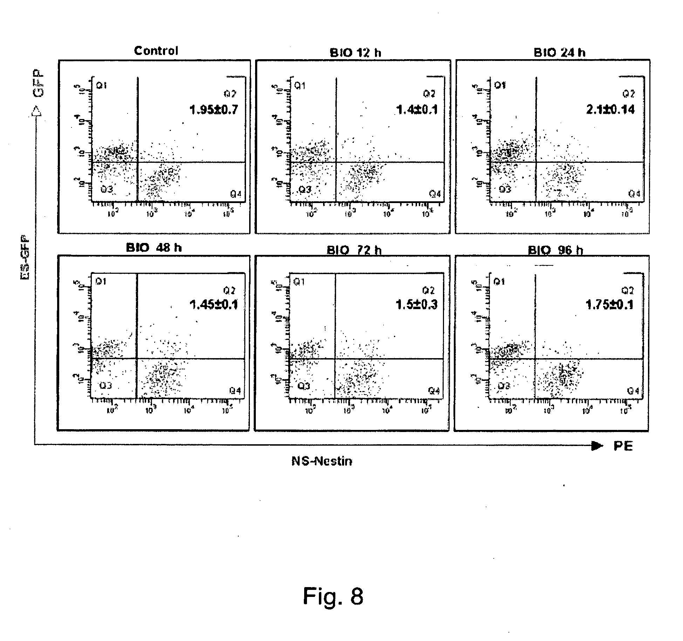

**Figure 8****. FACS analysis of the primary fusion products.** PEG fusions of ES-GFP cells with NS-Oct4-puro cells. Cells from fusion experiments were allowed to recover for 3 h after PEG fusion. The cells were then fixed and labelled for Nestin (expressed in NS cells only). The percentage of double-positive coloured (GFP/PE) cells is indicated in each panel.

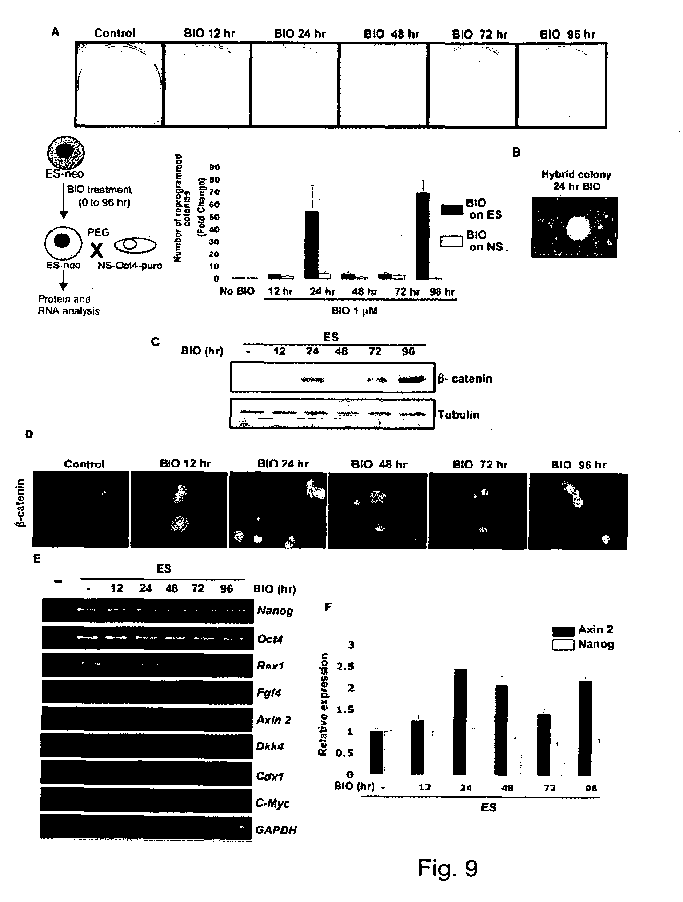

**Figure 9****. Periodic accumulation of β-catenin in ES cells via BIO inhibition of GSK-3 augments the reprogramming of NS cells without activation of stem-cell genes. (A)** Representative plates and quantification of hybrid colonies from PEG fusions between BIO-pretreated ES-Neo cells and non-treated NS-Oct4-puro cells, as indicated. Colonies were stained for the expression of AP and counted. The quantification also shows BIO-pretreated NS-Oct4-puro cells fused with non-treated ES-Neo cells (grey bars) (mean ±s.e.m.; n = 3). (**B**) Light microscopy image of a reprogrammed hybrid colony isolated from fusions with 24 h BIO-pretreated (24 h) ES-neo cells and NS-Oct4-puro cells (10x magnification). (**C**) Western blotting of protein extracts from BIO-pretreated ES cells in (A), as indicated. (**D**) Immunofluorescence for the visualization of β-catenin localization in ES cells from the BIO-pretreatments in (A), as indicated. (**E**) Semi-quantitative RT-PCR analysis of ES markers *(Nanog, Oct4, Rex1* and *Fgf4*) and β-catenin targets (*Axin2, Cdx1* and *c-Myc*) in total mRNA purified from BIO-pretreated ES cells in (A), as indicated. (**F**) Quantitative RT-PCR analysis *of Axin2* and *Nanog* transcripts in BIO-pretreated ES cells (mean ±s.e.m.; n = 3).

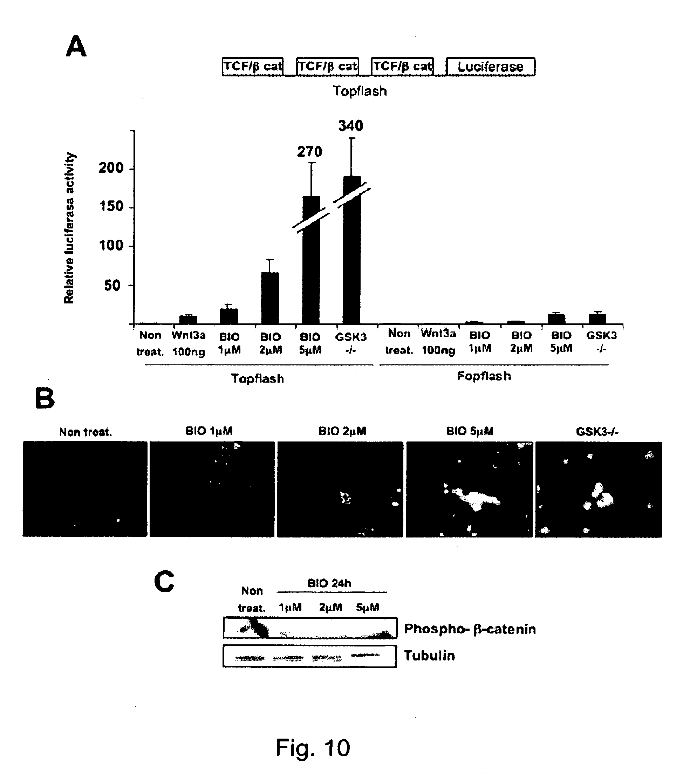

**Figure 10****. ES cells treated with increasing BIO concentrations show increased Topflash activation, increased β-catenin nuclear localization and decreased levels of phospho-β-catenin. (A)** Luciferase reporter assay of ES cells nucleofected with the reporter constructs (Topflash or Fopflash) and treated for 24 h with Wnt3a or different concentrations of BIO. GSK3-/- cells are used as the control as they induce very high Topflash activation. Each bar represents the average from three independent experiments (mean ±s.e.m.; n = 3). **(B)** β-catenin localization in ES cells treated for 24 h with BIO at different concentrations, as indicated **(C)** Western blotting of protein extracts of ES cells treated for 24 h with BIO at different concentrations, as indicated.

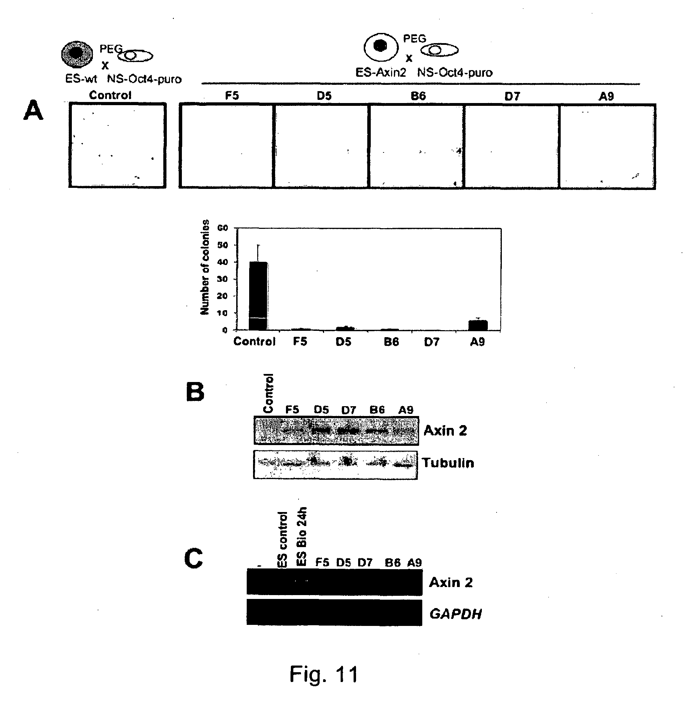

**Figure 11****. Axin2 over-expression in ES cells inhibits their reprogramming ability. (A)** Quantification (as number of colonies) of hybrid colonies from PEG fusions of ES-Axin2 clones (F5, D5, B6, D7, A9) with NS-Oct4-puro cells (mean ±s.e.m.; n = 3) **(B)** Western blotting of protein extracts from ES clones over-expressing Axin2 **(C)** RT-PCR analysis of Axin2 expression in ES treated for 24h with BIO and ES-Axin2 clones as indicated.

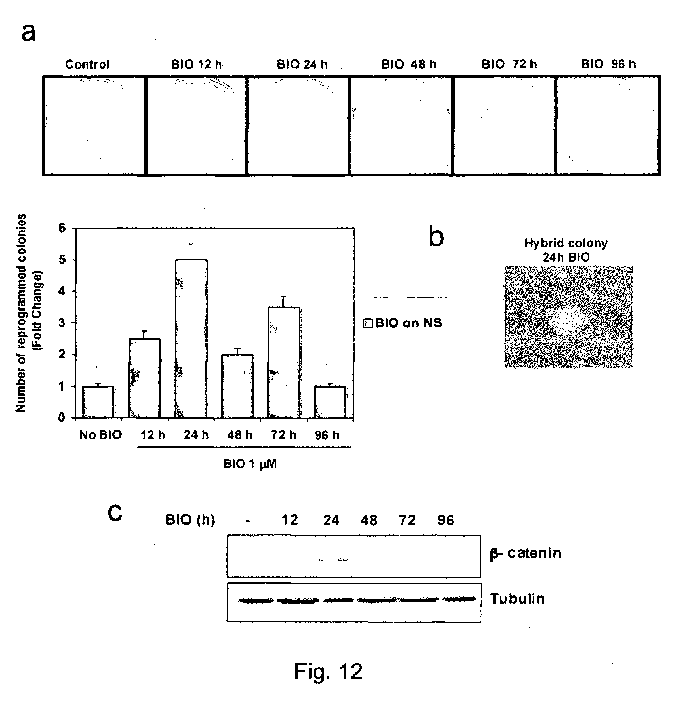

**Figure 12****. Treatment of NS-Oct4-puro cells with BIO before fusion with ES-neo cells results in poor enhancement of reprogramming.** (A) Plates containing fused hybrid colonies between BIO-treated NS-Oct4-puro cells and non-treated ES-neo cells. The clones were stained for the expression of AP and counted. The fold-change increases in the reprogrammed colonies are shown in the graph (mean ±s.e.m.; n = 3). (**B**) Example of a reprogrammed colony isolated from ES-neo cells fused with NS-Oct4-puro cells pretreated with BIO for 24 h. (**C**) Western blotting of total protein extracts of the NS-Oct4-puro cells at the indicated treatment times.

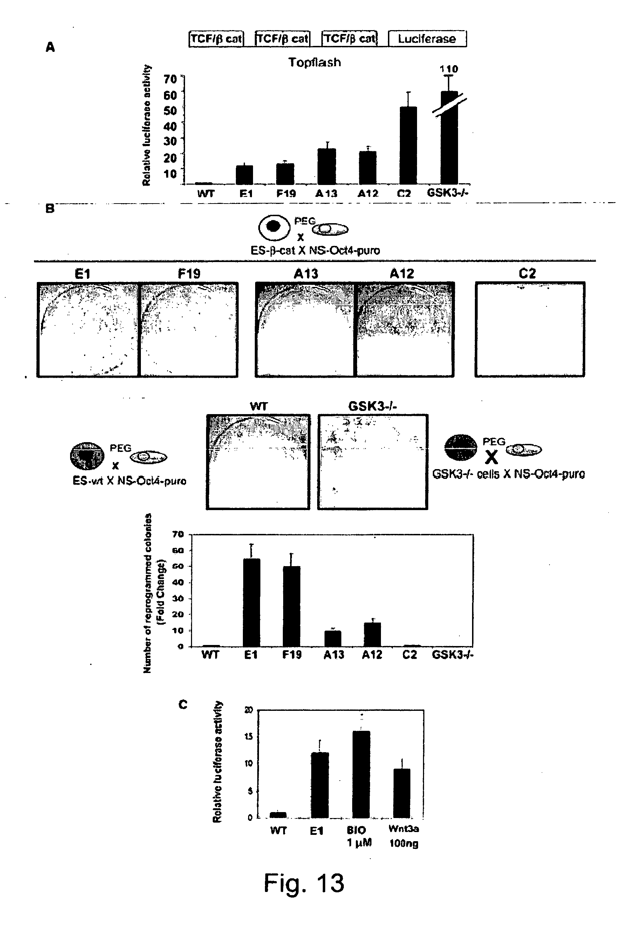

**Figure 13****. Low levels of stabilized β-catenin induce reprogramming of NS cells. (A)** Luciferase reporter assay of ES-β-catenin clones nucleofected with the Topflash reporter construct **(B)** Quantification (as fold-increases in the number of colonies) of hybrid colonies from PEG fusions of ES-β-catenin clones (E1, F19, A13, A12, C2) with NS-Oct4-puro cells, and from PEG fusion of wild-type ES or GSK3-/- cells with NS-Oct4-puro cells, as indicated (mean ±s.e.m.; n = 3). **(C)** Luciferase reporter (Topflash) assay of ES cells untreated and treated for 24 h with 1 µm BIO or 100 ng Wnt3a, and of the E1 clone (mean ±s.e.m.; n = 3).

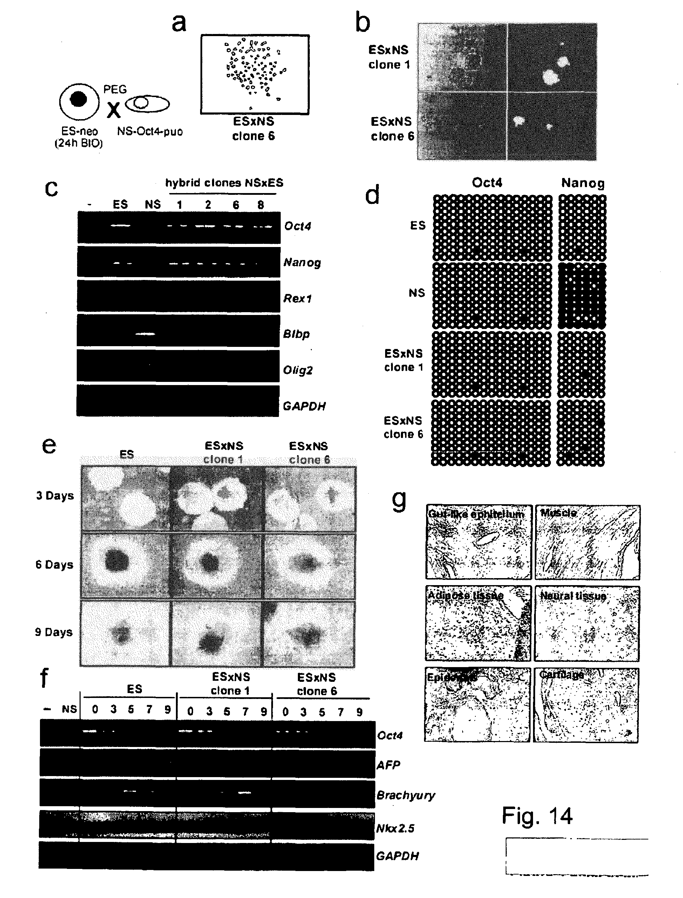

**Figure 14****. Isolated hybrids are pluripotent and can differentiate *in vitro* and *in vivo.* (A)** Normal karyotype and (**B**) GFP expression in different reprogrammed hybrid clones isolated from fusions of BIO-pretreated (24 h) ES-neo cells and NS-Oct4-puro cells. (**C**) RT-PCR analysis of *Oct4*, *Nanog*, *Rex1*, *Blbp* and *Olig2* in ES cells, NS cells and some of the hybrid clones, as indicated. (**D**) Bisulfite genomic sequencing of the promoter regions of *Oct4* and *Nanog* in ES cells, NS cells and hybrid clones. Open circles indicate non-methylated CpG dinucleotides; closed circles indicate methylated CpGs. (**E**) Morphology of embryoid bodies during differentiation, as indicated. (**F**) RT-PCR analysis of differentiation markers in ES cells, NS cells and hybrid clones, as indicated. (**G**) Hematoxylin and eosin staining of teratoma derived from cells of one reprogrammed clone transplanted subcutaneously into SCID mice.

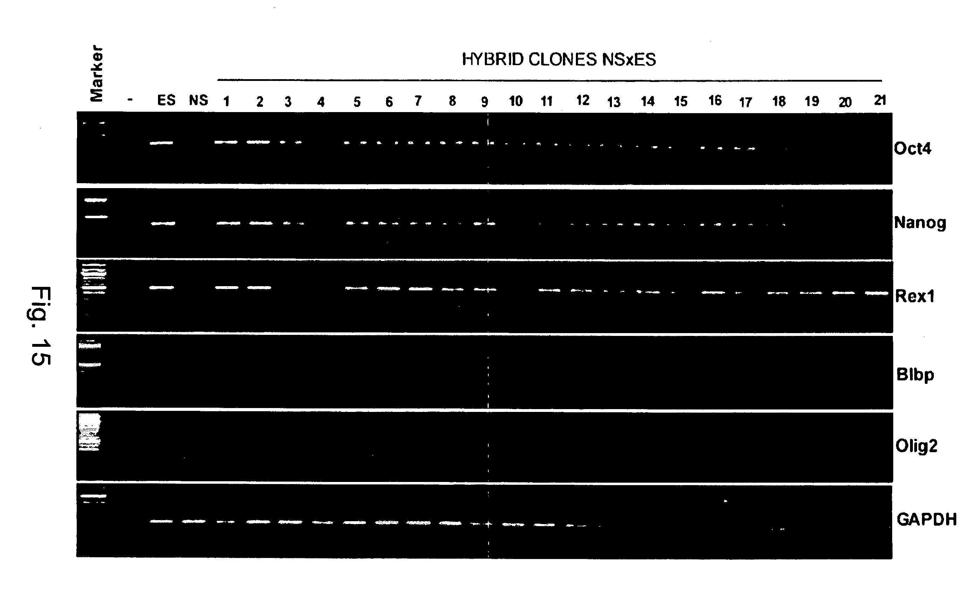

**Figure 15****. Analysis of the 21 different hybrid clones isolated from the fusion of NS-Oct4-puro cells with ES-neo cells that were pre-treated with BIO.** RT-PCR analysis of *Oct4, Nanog, Rex1, Blbp* and *Olig2* in ES-Neo, in NS-Oct4-puro and in the 21 hybrid clones.

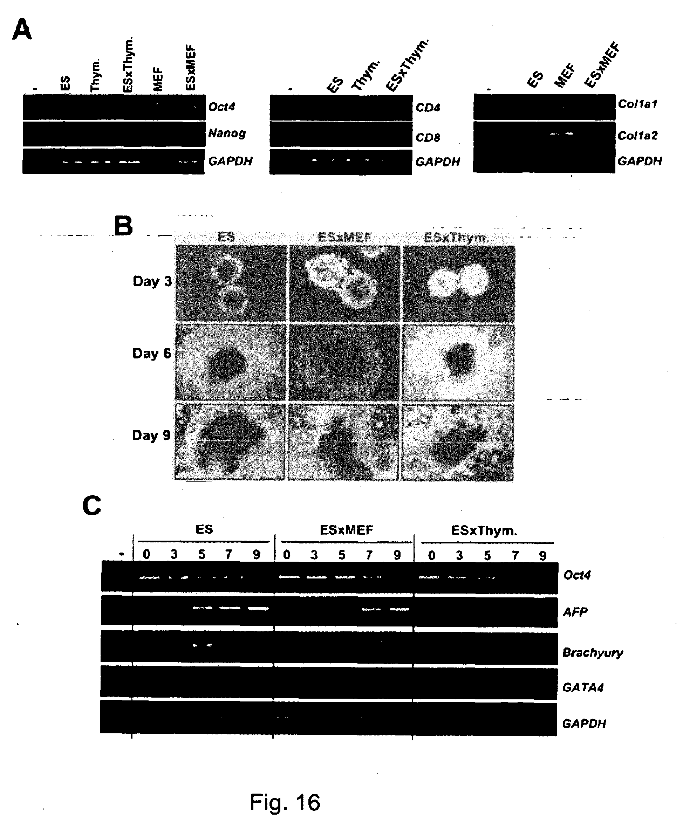

**Figure 16****. ES x MEFs and ES x thymocyte hybrids are pluripotent and can differentiate *in vitro.*** Reprogrammed pools isolated from fusions of ES-neo x MEFs-hygro and ES-hygro x Thymocytes-neo treated for 24 h with BIO. **(A)** RT-PCR analysis of Oct4, Nanog, CD4, CD8, Collal, Colla2 in ES cells, MEFs, thymocytes and hybrid clones, as indicated. **(B)** Morphology of embryoid bodies during differentiation, as indicated. **(C)** RT-PCR analysis of differentiation markers in ES cells and hybrid clones, as indicated.

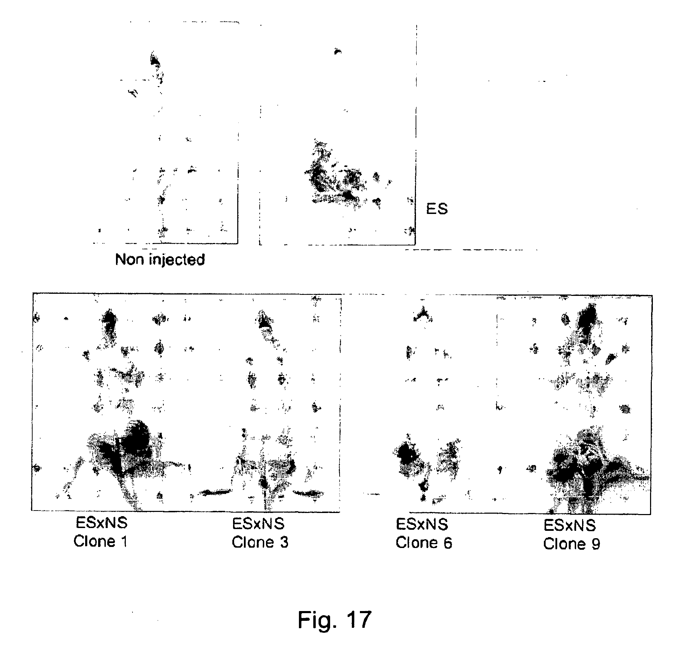

**Figure 17****. Teratoma formation of ES-neo x NS-Oct4-puro hybrid clones.** Hybrid cells (1.5x 10⁷) were injected subcutaneously in immuno-deficient (SCID) mice. Four weeks after injection, the teratomas were photographed before being collected and analyzed.

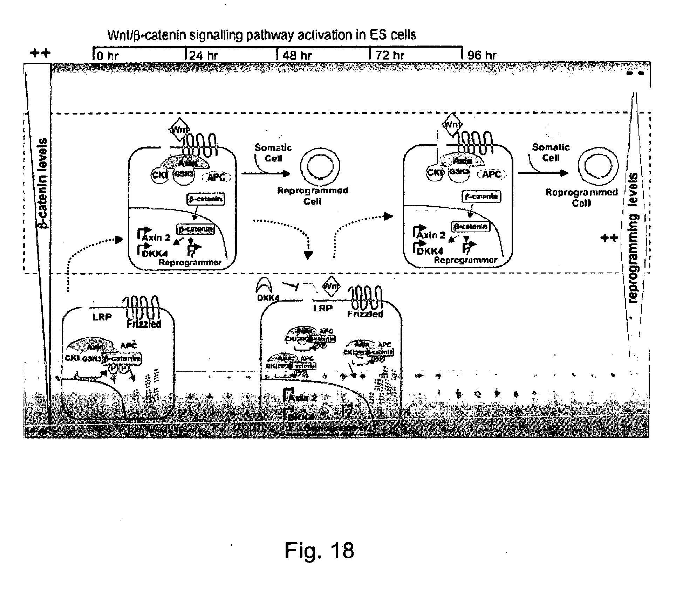

**Figure 18****. Periodic accumulation of β-catenin in ES cells enables them to reprogramme somatic cells after fusion. At** zero time (non-treated cells), the levels of β-catenin are low due to its degradation via the destruction complex. After 24 h of Wnt3a or BIO treatment, the Wnt/β-catenin signalling pathway is switched on, β-catenin is stabilized and enters the nucleus. Once in the nucleus, it activates several target genes, including a hypothetic unknown 'reprogrammer', a master factor that can drive the reprogramming of somatic cells after fusion. Accumulation of β-catenin also activates transcription of its inhibitors, Axin 2 and Dkk4. In the two subsequent time points, after 48 h and 72 h of Wnt3a or BIO treatment of ES cells, the high levels of Axin 2 increase the formation of the destruction complex and β-catenin is then phosphorylated and degraded. In addition, Dkk4 inhibits Wnt signalling by interacting with its receptor. Thus, ES cells cannot reprogramme somatic cells. Finally, 96 h of Wnt3a or BIO treatment of ES cells leads to activation of Wnt/β-catenin signalling, inactivation of the multiprotein destruction complex, β-catenin accumulation in the nucleus, transcription of the 'reprogrammer', and the consequent ability of the ES cells to reprogramme somatic cells after fusion. Very high and constant levels of β-catenin do not allow reprogramming, possibly because check point control mechanisms are activated and act as negative regulators.

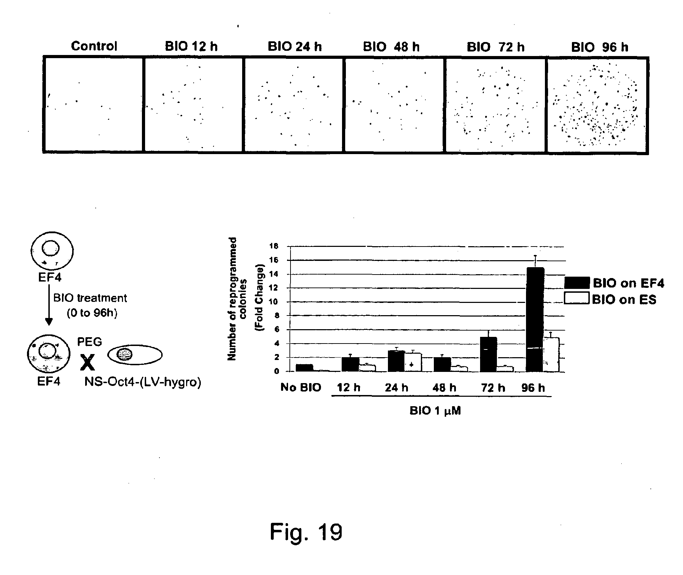

**Figure 19****. Nanog over-expression and Wnt pathway activation cooperate to enhance reprogramming of NS cells.** Representative plates and quantification (as fold-increases in the number of colonies) of hybrid colonies from PEG fusions between BIO-pretreated EF4 cells and non-treated NS-Oct4-(LV-Hygro) cells as indicated. Colonies were stained with giemsa and counted. The quantification also shows BIO-pretreated ES-Hygro cells fused with non-treated NS-Oct4-Puro cells (gray bars).

### Methods

### Cells

NS-Oct4-puro cells isolated from HP165 mice and carrying the regulatory sequences of the mouse *Oct4* gene driving GFP and puromycin resistance genes were a gift from Dr. A. Smith; they were cultured as previously described (Conti et al., 2005). Thymocytes were isolated from 6-8-week-old mice. Hygromycin-resistant MEFs were purchased at passage 3 (Millipore). ES-neo and ES-hygro cells were derived from E14Tg2a and transduced with the lentiviral pHRcPPT-PGK-Neomycin and pHRcPPT-PGK-Hygromycin vectors (Zito et al., 2005). ES GSK3 DKO cells were a gift from Dr. Doble, and they were cultured as previously described (Doble et al., 2007). ES cells were cultured on gelatin in knock-out Dulbecco's modified Eagle's medium (DMEM) supplemented with 20% foetal bovine serum (Hyclone), 1x non-essential amino acids, 1x GlutaMax, 1x 2-mercaptoethanol and 1,000 U/ml LIF ESGRO (Chemicon).

### Cell hybrids

ES x NS and ES x MEF PEG fusions: 1.2 x10⁶ NS cells or MEFs were plated into T25 flasks. After 2 h, 1.2 x10⁶ ES cells were plated onto them, allowing them to attach to the NS cells or MEFs. After a further 2 h, 1 ml 50% (w/v) PEG 1450 (Sigma; pre-warmed to 37 °C) was added for 2 min, and then the cells were washed three times with serum-free DMEM. Finally, they were cultivated with ES-cell complete medium without and with 1 µM BIO (Calbiochem), 100 ng/ml Wnt3a (R&D) or 50-100 ng/ml DKK1 (R&D). After 12 h, the cells were trypsinized and plated into gelatin+laminin-treated p100 dishes. For the ES x NS selection, puromycin was added after 72 h, while for the ES x MEF selection, hygromycin plus neomycin were added after 24 h.

ES x thymocyte fusions: the cells were fused in suspensions as previously described (Silva et al., 2006). After fusion, they were packed by centrifugation and the supernatant was discarded. The pelleted cells were resuspended in complete ES-cell medium and plated. The selection was carried out after 24 h, using hygromycin plus neomycin.

### Pretreated-ES x NS PEG fusions

1x10⁶ ES cells were plated in p100 plates and treated for 96, 72, 48, 24 and 12 h with 100 ng/ml of Wnt3a or 1 µM BIO. On the last day, the cells were trypsinized, counted, and plated on NS cells for fusions or harvested for RNA and protein analyses. PEG fusions and drug selection were performed as previously described.

### In-vitro differentiation of hybrid cells

The differentiation medium for the production of embryoid bodies consisted of ES-cell maintenance medium with no LIF supplementation. The cells were harvested by trypsinization, counted and propagated in hanging drops (400 single ES cells/30 µl initial drop) for 2 days before being transferred to 10 cm² bacterial dishes. At day 5, the embryoid bodies were transferred onto gelatinized p100 dishes.

### Teratoma production

Cells were trypsinized into single-cell suspensions and resuspended in phosphate-buffered saline to a concentration of 1.5 x10⁷ cells/ml. These cells were injected subcutaneously into the hind limbs of Fox Chase SCID mice using a 25-gauge needle (200 µl). Teratomas were collected after 4 weeks, and were fixed, embedded, sectioned and stained.

### Plasmid construction and stable ES cells

Mouse β-catenin mutated at serine 33, a kind gift of Dr. de la Luna, and mouse Axin2 (Chia and Costantini, 2005), a kind gift of Dr. Costantini, were sub-cloned in the empty pCAG-C1 vector produced in the laboratory which contained, in sequence order: CAG promoter, multicloning site, IRES, neomycin-resistance gene and polyA. Stable ES cell lines expressing β-catenin or Axin2 were isolated after nucleofection (Amaxa) of the two constructs and drug selection with 250 µg/ml neomycin.

### Western blotting

Western blotting was performed as previously described (Zito et al., 2007). The primary antibodies used were: anti-β-catenin, clone 14 (BD Biosciences); anti-phospho-β-catenin, S33/S37/T41 (#9561 Cell Signaling Technologies); anti-conductin sc-20784 (Santa Cruz); anti-APC, sc-895 (Santa Cruz); and anti-β-tubulin, clone D66, T0198 (Sigma).

### Immunostaining

ES cells were washed twice with phosphate-buffered saline (PBS), fixed with 4% paraformaldehyde and blocked in 5% goat serum for 40 min. The cells were incubated with anti-β-catenin, clone 14 (BD Biosciences) for 2 h and with an anti-mouse antibody conjugated with fluorescein for 1 h. The cells were then washed and mounted on slides with a few drops of Vectashield with DAPI (Vector Laboratories).

### Transient transfections and luciferase activity

ES cells were co-transfected by nucleofection (Amaxa) with the Topflash reporter construct driving firefly luciferase cDNA and a pRL-CMV driving constitutive expression of renilla cDNA for normalization. Treatments with Wnt3a and BIO were carried out the day after the nucleofection and cells were lysed with 1x passive reporter lysis buffer. Firefly and renilla reporter activities were measured using a 96-well-based luminometer and were detected as per manufacturer instructions (Promega Dual-Light system),

### Semiquantitative RT-PCR analysis

For RT-PCR, the total RNA was extracted from ES cells and from embryoid bodies (200 embryoid bodies for each clone and for each differentiation time point) using the RNeasy Kit (Qiagen), and the cDNA was generated using superscript III (Invitrogen). The primers used were:
- Oct4:: forward, GGCGTTCTCTTTGGAAAGGTGTTC (SEQ ID No. 1);
reverse, CTCGAACCACATCCTTCTCT (SEQ ID No. 2).
- Nanog:: forward, AGGGTCTGCTACTGAGATGCTCTG (SEQ ID No. 3);
reverse, CAACCACTGGTTTTTCTGCCACCG (SEQ ID No. 4).
- Rex1:: forward, GCCCTCGACAGACTGACCCTAA (SEQ ID No. 5);
reverse, CTTCCTCAGGGCGGTTTTACCC (SEQ ID No. 6).
- Fgf4:: forward, GACTACCTGCTGGGCCTCAA (SEQ ID No. 7);
reverse, CGACACTCGGTTCCCCTTCT (SEQ ID No. 8).
- Olig2:: forward, GCGTGGGTATCAGAAGCACT (SEQ ID No. 9);
reverse, CCAGTCGGGTAAGAAACCAA (SEQ ID No. 10).
- Blbp:: forward, GGGTAAGACCCGAGTTCCTC (SEQ ID No. 11);
reverse, ATCACCACTTTGCCACCTTC (SEQ ID No. 12).
- Axin2:: forward GGGAGCAGTTTTGTGGCAGCA (SEQ ID No. 13);
reverse, AGGGTCCTGGGTAAATGGGTGAG (SEQ ID No. 14)
- C-Myc:: forward, TGCCGCCCACTCTCCCCAACC (SEQ ID No. 15);
reverse, CCGCCGCCGTCATCGTCTTCC (SEQ ID No. 16).
- Brachyury:: forward, TGCTGCCTGTGAGTCATA (SEQ ID No. 17);
reverse, ACAAGAGGCTGTAGAACATG (SEQ ID No. 18).
- Nkx2.5:: forward, GCTCTCCTGCTTTCCCAGC (SEQ ID No. 19);
reverse, CTCCCATCCCTACTGCCTTCTGCAGC (SEQ ID No. 20).
- AFP:: forward, TCCCTCATCCTCCTGCTA (SEQ ID No. 21);
reverse, GCACATTCTTCTCCGTCAC (SEQ ID No. 22).
- Cdx 1:: forward TCTACACAGACCACCAACGC (SEQ ID No. 23)
reverse AGAAACTCCTCCTTGACGGG (SEQ ID No. 24).
- FGF5:: forward, AAAGTCAATGGCTCCCACGAA (SEQ ID No. 25);
reverse, CTTCAGTCTGTACTTCACTGG (SEQ ID No. 26).
- GAPDH:: forward, ACTCCCACTCTTCCACCTTC (SEQ ID No. 27);
reverse, TCTTGCTCAGTGTCCTTGC (SEQ ID No. 28).
- CD4:: forward, CTGCGAGAGTTCCCAGAAGA (SEQ ID No. 33);
reverse,TTCCTGTTCTCCAGCTCACA (SEQ ID No. 34);
- CD8:: forward, GCTCAGTCATCAGCCAACTCG (SEQ ID No. 35);
reverse, ATCACAGGCGAAGTCCAATC (SEQ ID No. 36);
- Colla1:: forward, GCAGACGGGAGTTTCTCCTC (SEQ ID No. 37);
reverse, TCAAGCATACCTCGGGTTTC (SEQ ID No. 38);
- Col1a2:: forward, CGACTAAGTTGGAGGGAACG (SEQ ID No. 39);
reverse, CTTTGTCCACGTGGTCCTCT (SEQ ID No. 40).
- GATA4:: forward, GTCGTAATGCCGAGGGTGA (SEQ ID No. 41);
reverse, TCCTTCCGCATTGCAAGAG (SEQ ID No. 42).

### Bisulfite genomic sequencing

Bisulfite treatment was performed using the Epitect Bisulfite Kit (Qiagen), according to the manufacturer recommendations. The amplified products were cloned into pCR2.1-TOPO (Invitrogen). Ten randomly selected clones were sequenced with the M13 forward and M13 reverse primers for each gene, as follows:
- MeNanog:: forward, GATTTTGTAGGTGGGATTAATTGTGAATTT (SEQ ID No. 29);
reverse, ACCAAAAAAACCCACACTCATATCAATATA (SEQ ID No. 30).
- MeOct4:: forward, GGTTTTTTAGAGGATGGTTGAGTG (SEQ ID No. 31);
reverse, TCCAACCCTACTAACCCATCACC (SEQ ID No. 32).

### Quantitative RT-PCR

RNA isolation and reverse transcription were carried out as described for semiquantitative RT-PCR. The template for each PCR reaction was the cDNA obtained from 16 ng total RNA in a 25-µl reaction volume. Platinum SYBR green qPCix-UDG (Invitrogen) was used with an ABprism 7000 real-time PCR machine, according to the manufacturer recommendations. The primers used were:
- NanogRT:: forward, AACCAAAGGATGAAGTGCAAGCGG (SEQ ID No. 43);
reverse, TCCAAGTTGGGTTGGTCCAAGTCT (SEQ ID No. 44).
Axin2 (purchased, SuperArray Bioscience Corporation).

### FACS analysis

ES-GFP cells were treated or not with BIO for 12, 24, 48, 72 and 96 h and subsequently PEG fused with NS-Oct4-puro cells. Hybrids were trypsinized, counted, resuspended in PBS (1x10⁶ cells per point) and centrifuged. Then, 0.1 ml of 4% paraformaldehyde solution was added to the cells and they were incubated for 15 min. Blocking solution (0.1% Triton, 10% goat-serum, in PBS) was subsequently added and the cells incubated for 1 h. The cells were then labelled with α-nestin (1:100) (ab6142, Abcan) overnight. The day after, the cells were incubated with the secondary antibody conjugated with PE fluorocrome (1:50) (ab7002 Abcam) for 1 h. FACS analyses were performed using a BDBiosciences FACSAria cytometer.

### In-vitro differentiation of hybrid cells

The differentiation medium for the production of embryoid bodies consisted of ES-cell maintenance medium with no LIF supplementation. The cells were harvested by trypsinization, counted and propagated in hanging drops (400 single ES cells/30 µl initial drop) for 2 days before being transferred to 10 cm² bacterial dishes. At day 5, the embryoid bodies were transferred onto gelatinized p100 dishes.

### Nanog expression

### Cells

NS-Oct4-(LV-Hygro) cells were derived from NS-Oct4-puro and transduced with the letiviral pHRcPPT-PGK-Hygromycin vector. EF4 cells carrying the CAG promoter driving Nanog and Puromycin resistance genes were a gift of Dr. I. Chambers. Cells were cultured as previously described (Chambers et al., 2003).

### Cell hybrids

NSxES-Hygro or NS-HygroxEF4 cells PEG fusions were done as previously described. For the NSxES-Hygro and NSxEF4 selection, Puromycin plus Hygromycin was added after 72 h.

### Results

The binding of Wnt ligands to the Frizzled receptor results in inactivation of a multiprotein complex composed of glycogene synthase kinase-3 (GSK-3), Axin1, Axin2 and adenomatous polyposis coli (APC). Inactivation of GSK-3 leads to a decrease in β-catenin phosphorylation, which then escapes ubiquitin/ proteosome-mediated degradation (Willert and Jones, 2006). As a consequence, non-degraded β-catenin accumulates in the cytoplasm and translocates into the nucleus, leading to transcriptional activation of a plethora of target genes (Hoppler and Kavanagh 2007). Wnt3a-cultured embryonic stem (ES) cells can self renew and remain totipotent due to the accumulation of β-catenin (Anton et al., 2007). In addition, both inactivation of the *GSK-3a* and *GSK-3*β homologs and mutations in *APC* severely compromise the ability of ES cells to differentiate (Doble et al., 2007, Kielman et al., 2002). Of note, the regeneration of hair follicles and of cardiovascular progenitors is Wnt dependent (Ito et al., 2007, Qyang et al., 2007). These observations prompted the authors to determine if Wnt/β-catenin signaling is a pathway that can drive reprogramming of somatic cells.

### Activation of the Wnt/β-catenin signalling pathway enhances reprogramming of somatic cells after fusion

The authors used polyethyleneglycol (PEG) to generate fusion hybrids between ES cells constitutively expressing the *NeoR* gene, and mouse neural stem (NS)-Oct4-puro/GFP cells (Silva et al., 2006) expressing the *PuroR* gene and green fluorescent protein (GFP) under the control of the *Oct4* (*Pou5f1*) regulatory element that is only active in pluripotent cells (Yeom et al., 1996). Immediately after fusion, the cells were cultured in gelatin (without feeders that express different Wnt isoforms (Dravid et al., 2005; Wiese et al., 2006)) for 12, 24, and 48 h (Figure 1A) in the presence of an optimized concentration of Wnt3a (100 ng/ml; Figure 2A). Hybrids were selected in ES-cell medium supplemented with puromycin; under these culture conditions only reprogrammed clones can grow. ES-cell medium induces differentiation and growth arrest of NS cells, and thus of non-reprogrammed hybrids too. Furthermore, puromycin provides additional selection for the reprogrammed clones (Figure 3).

The resistant colonies were stained for the expression of alkaline phosphatase (AP), an ES-cell marker, and counted (Figure 1A). These cells had been reprogrammed, as they retained a rounded ES-cell-like phenotype and expressed AP and Oct4-puro/GFP (Figure 1A, B). The authors selected up to 20-fold more reprogrammed clones with respect to the control after 24 h of Wnt3a culturing. The number of reprogrammed clones decreased after 48 h of treatment, indicating that prolonged Wnt3a culturing might diminish the reprogramming efficiency.

These data clearly showed that time-dependent Wnt3a treatment greatly enhances the ability of ES cells to reprogramme NS cells.

In support of these data, a very high number of AP-positive reprogrammed clones (between 30 and 300) showing an ES-cell-like phenotype were obtained when mouse embryonic fibroblasts (MEFs) expressing different Wnt isoforms (Dravid et al., 2005; Wiese et al., 2006) were fused with ES cells. This number increased up to 5-fold when these hybrids were cultured in the presence of Wnt3a for 48 h (Figure 1C). Finally, to confirm that activation of the Wnt/β-catenin pathway enhances somatic cell reprogramming, the authors inhibited this pathway with Dkk1, an LRP6 receptor inhibitor (Logan and Nusse, 2004). The striking decrease in the number of reprogrammed, AP-positive clones (up to 6-fold; Figure 1C) demonstrates that the Wnt/β-catenin signalling pathway stimulates the reprogramming of somatic cells.

### Transient inhibition of GSK3 in ES cells enhances their ability to reprogramme different somatic cells.

To confirm that cell reprogramming is augmented through the activation of the canonical Wnt/β-catenin signalling pathway, the authors decided to transiently inhibit GSK-3 by addition to the culture medium of the selective GSK-3 inhibitor 6-bromoindirubin-3'-oxime (BIO) (Meijer et al., 2003). This inhibition of GSK-3 mimics the activation of the pathway via Wnt3a, as it promotes the accumulation of β-catenin in the cell nucleus (Meijer et al., 2003) and maintains the pluripotency of stem cells (Sato et al., 2004). Initially, to exclude a direct role of BIO in enhancing clonogenicity and/or viability of ES cells and hybrid clones, the authors plated scaled numbers of ES cells or hybrids in BIO-containing medium. This demonstrated that the clonogenicity and viability of both ES and hybrids cultured in the absence and presence of BIO, respectively, were equal (Figure 4A, B). In addition, since the Wnt/β-catenin signalling pathway is regulated by an inhibitory feedback loop (Ueno et al., 2007), the authors tested different concentrations of BIO to determine the concentration that mimics this physiological regulation most closely: addition of 1 µM BIO led to the relatively limited activation of the Topflash reporter gene (a vector harbouring β-catenin binding sites fused to luciferase) and resembled the level of activity upon Wnt3a stimulation (Figure +10A). In addition, higher BIO concentrations were slightly toxic to NS cells (data not shown). Thus, the authors used 1µM BIO as standard under the remaining experimental conditions.

The authors then analyzed the effects of BIO treatment over time. PEG-fused ES-neo x NS-Oct4-puro cells were cultured for 12, 24, and 48 h with this optimized concentration of BIO (1 µM; Figure 2B). After 24 h of treatment, the authors obtained up to 70-fold more reprogrammed clones (from 200 to 1,500 total clones), with respect to the control (Figure 5A). Reprogramming was dependent on the time of BIO stimulation, with a decreased number of reprogrammed clones after 48 h of treatment. The selected hybrids were reprogrammed as they showed an ES-cell-like phenotype, were AP-positive, and expressed GFP-puro (Figure 5A, B). However, to exclude a role of BIO as an enhancer of cell fusion, the authors performed further control experiments. BIO did not enhance PEG-mediated fusion of ES cells, as there was only a slight increase in the number of clones after the fusion of BIO-pretreated ES-neo cells with ES-Hygro cells (Figure 6A). In the fusion of ES x ES cells, the authors could only select for hybrids and not for reprogrammed clones, and the number of clones obtained was comparable. Furthermore, to better exclude the possibility that BIO has a fusogenic activity on different cell types, the authors PEG-fused ES-neo x NS-Oct4-puro cells for 3 h and then re-plated the hybrids at a dilution such that the cells were not in contact with each other. Thus, BIO was added to the cell medium for 12, 24, 48 and 72 h (Figure 6B). The hybrids were formed before the addition of BIO and they could not increase further since the cells were not touching each other. Nevertheless, however, reprogrammed clones were selected with a pick at 24 h of BIO treatment. (Figure 6B). This further confirms that BIO enhances the reprogramming, and not the fusion, of cells.

Inhibition of GSK-3 with BIO also strikingly increased the numbers of reprogrammed colonies when MEFs and thymocytes were fused with ES cells. PEG-fused hybrids of ES-neo x MEF-hygro and ES-hygro x thymocyte-neo cells were cultured in BIO for 12, 24 and 48 h, and double-drug selected. There were up to 20-fold (between 200 and 1,500 total clones) and 9-fold (between 20 and 100 total clones) increases, respectively, in the numbers of reprogrammed selected clones, with respect to their controls (fusions in the absence of BIO; Figure 5C, E). Again, the reprogramming efficiency was lower with increased treatment time with BIO. All of the selected clones showed an ES-like phenotype and were AP positive (Figure 5C-F).

These data demonstrate that transient inhibition of GSK-3 greatly enhances the ability of ES cells to reprogramme somatic cells. Furthermore, this reprogramming occurs with variable timing across different cell types.

### Cyclic stabilization of β-catenin in ES cells enhances their ability to reprogramme NS cells

The authors then asked whether the activation of the Wnt/β-catenin pathway in ES cells would strengthen and enhance their reprogramming ability. Thus, the authors investigated whether an enhancement of reprogramming occurs when ES and/or NS cells are cultured with Wnt3a or BIO before their fusion. ES cells were thus cultured in Wnt3a or BIO for 12, 24, 48, 72 and 96 h, and fused with NS-Oct4-puro cells grown in the absence of BIO. Up to 15-fold (about 600 total clones) or 80-fold (about 2,000 total clones) increases in the numbers of reprogrammed clones were seen when ES cells were pre-cultured for 24 h and 96 h with Wnt3a or BIO, respectively (Figures 7A and 9A); the selected clones showed an ES-cell-like phenotype, were AP positive, and expressed *Oct4* (Figures 7A and 9A, B). In contrast, there was poor enhancement of NS reprogramming with the ES cells cultured with Wnt3a or BIO for 12, 48 and 72 h. To ensure that pre-treatment of ES cells with BIO before fusing them with NS-Oct4-puro cells did not increase fusion, PEG-fused cells were cultured for 3 h, an insufficient time for reprogramming to occur, and FACS analysis was carried out. The same number of hybrid clones were seen whether the ES cells were pre-treated or not with BIO before the fusion, thus excluding BIO-dependent, enhanced fusogenic activity (Figure 8). The authors thus asked why there was a parabolic wave of reprogramming through the periods of Wnt3a and BIO treatment. This appeared to be due to β-catenin accumulation in both Wnt3a-and BIO-treated cells at 24 h and 96 h (Figures 7B and 9C), and to its consequent localization in the cell nucleus at these two times of treatment (Figures 7C and 9D). These data show that reprogramming can be triggered only after the cyclic accumulation of β-catenin. While 12 h of Wnt3a or BIO treatment was not sufficient to accumulate enough β-catenin to trigger reprogramming, 24 h (and 96 h) of Wnt3a or BIO pretreatment of ES cells resulted in the accumulation of β-catenin; at the same times, *Axin2, Dkk4* and APC were also expressed, leading to a negative-feedback loop on the Wnt/β-catenin pathway (Bazzi et al., 2007; Doble et al., 2007; Jho et al., 2002; Lustig et al., 2002; Niida et al., 2004; Yan et al., 2001) that affected the subsequent 48 h and 72 h time points (Figures 7B, D, E and 9C, E, F, and data not shown). The wave of β-catenin accumulation is similar in both Wnt3a- and BIO-treated ES cells. This might be due to the relatively low concentration of BIO used here, which might not inhibit GSK-3 activity fully, thus resembling the feedback loop regulation in Wnt3a-treated cells. Indeed, with 1 µM BIO, the amount of phospho-β-catenin in ES cells was higher with respect to the cells treated with increased BIO concentrations, and moreover, the nuclear accumulation of the non-phosphorylated form was rather low. This leads to the conclusion that GSK-3 was not fully inhibited by 1 µM BIO (Figure 10B, C). To confirm that the increased expression of Axin2 was preventing the reprogramming, the authors generated stable ES clones over-expressing Axin2 (ES-Axin2) and fused them with NS-Oct4-puro cells. No reprogrammed clones were selected after fusion with five different ES-Axin2 clones (Figure 11). In conclusion, activation of Wnt/β-catenin signalling is a key mechanism for the reprogramming of somatic cells, and only when β-catenin accumulates in ES cells to a certain threshold can these cells reprogramme NS cells after fusion.

Remarkably, Wnt3a- and BIO-treated ES cells expressed the same levels of the pluripotent genes *Nanog, Oct4, Rex1* and *Fgf4* at all of the treatment times, whereas expression of the β-catenin-dependent genes *Cdx1, Axin2* and *Dkk4* cycled according to the accumulation of the β-catenin protein itself (Figures 7D, E and 9E, F). Of note, although *c-Myc,* a known β-catenin target gene (He et al., 1998), has previously been shown to enhance reprogramming when over-expressed together with three other genes (Takahashi and Yamanaka, 2006), here it was constantly expressed under Wnt3a and BIO treatments. These data suggest that pretreatment of ES cells with Wnt3a or BIO results in the activation of the Wnt/β-catenin signalling pathway and expression of specific β-catenin target genes that, in turn, stimulate the reprogramming of somatic cells. This pathway is independent of *Nanog, Oct4, Rex1, Fgf4* and *c-Myc;* indeed, these genes were expressed at the same levels throughout the different Wnt3a and BIO treatments.

The alternative of pretreatment of NS cells with BIO led to β-catenin accumulation at 24 h and 72 h, but provided poor enhancement of reprogramming, with only a slight increase in the number of ES-cell-like, AP-positive reprogrammed clones (Figure 9A and Figure 12). Thus, the Wnt/β-catenin signalling pathway drives a remarkable enhancement of reprogramming of somatic cells only when it is activated in ES cells.

Next, the authors wanted to better characterize the mechanism of activation of reprogramming and to ensure that β-catenin is a key factor in the reprogramming mechanism of somatic cells. The authors asked whether ES cells expressing different levels of β-catenin have different reprogramming abilities. For this, the authors generated stable ES clones over-expressing different levels of the stabilized β-catenin (ES-β-catenin) harbouring a mutation in one of its destruction-complex-dependent phosphorylation sites. The authors selected five clones that activated expression of the Topflash reporter gene with different degrees of activity, with E1 and F19 being the least active, A13 and A12 with intermediate activity, and C2 the most active and most similar to GSK-3-/- ES cells, which accumulate a constant and high amount of β-catenin (Doble et al., 2007) and greatly activate Topflash (Figure 13A). A very high number of reprogrammed clones (up to a 55-fold increase) were generated after fusion of the E1 and of F19 clones with NS-Oct4-puro cells, an intermediate number were generated upon fusion with the A13 and A12 clones (up to an 18-fold increase), and almost no reprogrammed clones were selected after fusion of the C2 clone and of GSK3-/- cells (Figure 13B). Of note, the E1 clone activated the Topflash reporter gene to a similar extent to that seen for 1 µM BIO or Wnt3a 24h-treated cells (Figure 13C), leading to the conclusion that these cells express similar levels of active β-catenin, and therefore have comparable reprogramming capacities. These data clearly demonstrate that reprogramming occurs only when the levels of β-catenin are neither too low nor too high; the levels of β-catenin need to be within an intermediate physiological level, which the E1 and F19 clones and both Wnt3a- and 1 µM BIO-treated ES cells have.

### Isolated clones are reprogrammed and can differentiate in vitro and in vivo

To confirm pluripotency, and thus the ES-cell-like phenotype, 21 different reprogrammed clones were isolated from the fusion of ES cells pre-treated with BIO for 24 h with NS-Oct4-puro cells. The clones were all tetraploids (Figure 14A), were GFP positive, and expressed *Oct4, Nanog* and *Rex1* after several passages (Figure 14B). In contrast, the neural *Blbp* and *Olig2* genes were shut off (Figure 14C and Figure 15). Likewise, ES x thymocytes and ES x MEFs reprogrammed hybrids expressed *Oct4* and *Nanog* and lost expression of *CD4* and *CD8,* and *Colla1* and *Colla2* specific markers, respectively (Figure 16A). Furthermore, bisulfite genomic sequence analysis revealed that in the ES x NS reprogrammed clones, the CpG islands of the *Nanog* and *Oct4* promoters were highly demethylated and were comparable to ES cells, suggesting that these two loci had undergone massive epigenetic modifications; in contrast, the same CpGs were highly methylated in parental NS cells (Figure 14D). These data clearly show that the hybrids isolated from the fusions with ES cells pre-treated with BIO had been reprogrammed.

Next, to determine the differentiation ability of the reprogrammed clones, the authors induced those of ES x NS, ES x thymocytes and ES x MEFs to spontaneous differentiation. Embryoid bodies were formed: ball-shaped structures were clearly visible after 3 days and underwent differentiation after 6 and 9 days (Figure 14E and Figure 16B). Expression of *Oct4* (pluripotent stem cell), *AFP* (endoderm), *Brachyury* (mesoderm), *Nkx2.5* (cardiac muscle) and *GATA4* (cardiac muscle) were seen at 3, 5, 7 and 9 days of differentiation, with their expected timing (Boheler et al., 2002), and as comparable to ES cells (Figure 14F and Figure 16C). Furthermore, some of the clones started beating spontaneously, demonstrating their differentiation into cardiomyocytes

Finally, to test the differentiation ability of ES x NS hybrids *in vivo,* the authors transplanted five different clones subcutaneously in immuno-deficient (SCID) mice. Three weeks after injection, teratomas were seen (Figure 17). Histological examination showed the presence of gut-like epithelial tissue (endoderm), striated muscle and adipose tissue (mesoderm), neural tissue, epidermis, and cartilage (ectoderm) (Figure 14G).

These data clearly demonstrate the differentiation ability of the reprogrammed clones, both *in vitro* and *in vivo.*

The authors' data demonstrate that activation of the Wnt/β-catenin signaling pathway without over-expression of any of the known ES-cell genes can control reprogramming of somatic cells. Indeed, in a system with both Nanog overexpression and Wnt pathway activation, the reprogramming of NS cells is enhanced even further (Fig. 19), indicating that these two pathways can cooperate, but remain distinct. The authors have shown that the cycling accumulation of β-catenin determines reprogramming, mimicking a physiological scenario that might occur *in vivo.* In contrast, stable gain-of-function of β-catenin may result in tumor formation (Katoh et al., 2007) or the inability of reprogrammed cells to differentiate after fusion (Doble et al., 2007, Kielman et al., 2002), whereas its loss of function may result in failure of reprogramming (Haegel et al., 1995).

Finally, the authors postulate that a novel gene that is probably an epigenetic factor that is not constitutively expressed in ES cells can be activated via Wnt/β-catenin signaling, to switch on a cascade of events that triggers reprogramming of the genome of somatic nuclei. Our data lead us to the highly speculative and provocative hypothesis that regeneration of injured tissues might be kicked off via "commanding" stem cells that are activated via the Wnt/β-catenin pathway and that are able to reprogram somatic cells after fusion.

### Discussion

The authors have shown here that transient activation of the Wnt/β-catenin signalling pathway leads to a cyclic accumulation of β-catenin in ES cells that enables them to reprogramme somatic cells after fusion. When, and only when, a specific level of β-catenin is stabilized and translocates into the nucleus, are ES cells able to reprogramme differentiated cells; in contrast, when β-catenin is degraded, reprogramming does not occur. The different levels of β-catenin over time are likely to be due to the expression of some of its inhibitory targets, such as Axin2 and Dkk4, which stabilize the destruction complex and inhibit Wnt receptors, respectively. A negative feedback loop of the Wnt/β-catenin signalling pathway has been shown in differentiating ES cells, which can result in both positive and negative effects on cardiogenesis (Ueno et al., 2007). Indeed, the authors have shown that over-expression of Axin2 in ES cells impairs their reprogramming ability. The periodic accumulation of active β-catenin or low amounts of a stabilized β-catenin mutant are thus essential in the reprogramming process; in contrast, stable and high levels of β-catenin affect the ability of ES cells to reprogramme somatic cells (Figure 7).

Why is the level and timing of β-catenin accumulation so important for reprogramming efficiency? A stable gain-of-function form of β-catenin can lead to tumour formation (Katoh and Katoh, 2007). In addition, GSK-3-/- ES cells that express stable β-catenin levels cannot differentiate (Doble et al., 2007; Kielman et al., 2002), and the authors have shown that these cells, as ES clones harbouring high β catenin activities, are not able to reprogramme somatic cells. On the other hand, a loss-of-function of β-catenin has severe defects in mouse development at the gastrulation stage (Haegel et al., 1995; Huelsken et al., 2000) and P-catenin-/- ES cells do not express some stem-cell markers (Anton et al., 2007). Furthermore, the authors have shown here that basal levels of active β-catenin in ES cells can activate reprogramming only very poorly.

Thus, both loss-of-function and gain-of-function of β-catenin are highly toxic for cells, with grave effects on their differentiation ability and capacity to induce reprogramming. In contrast, transient Wnt/β-catenin signalling activation is regulated by the negative-feedback loop that tunes the level of β-catenin, resulting in waves of its accumulation up to a specific threshold, and the induction of reprogramming.

Once in the nucleus, β-catenin activates different target genes (Katoh and Katoh, 2007). Here, the authors postulate that one or more factor(s), the "reprogrammer(s)" that are not constitutively expressed in ES cells, are transcribed upon β-catenin binding, and switch on a cascade of events that triggers the reprogramming of the genome of the somatic nucleus. This is suggested also by the finding that reprogramming occurs with variable timing across various cell types, which leads to the prediction that β-catenin initiates a complex programme of events that can last for different times in different cell types.

Reprogramming could be due to trans-acting factors that can trigger transcription and/or suppression of somatic-nucleus-specific genes, implying that these factors must be present constantly to maintain the non-differentiated phenotype. Alternatively, the induction of a new heritable epigenetic modification of the somatic nucleus could switch a programme, resulting in massive chromatin modifications. Whatever the mechanism is, the transcription of the reprogrammer(s) itself are probably finely tuned by checkpoint controls that regulate its concentrations; indeed, high levels of stabilized β-catenin in GSK3-/- or C2 cells that had skipped destruction complex inactivation, abolished the reprogramming ability of ES cells, leading us to envisage that there are alternative checkpoint systems that control the concentrations of the reprogrammer(s) that might come into action.

In human and mouse ES cells, the polycomb group (PcG) proteins show transcriptional repression of the expression of developmental genes, which would otherwise promote differentiation (Boyer et al., 2006b; Lee et al., 2006). The chromatin structure of many of these PcG-regulated genes contain 'bivalent-domain' modifications that consist of both inhibitory histone H3 lysine 27 methylation and activating histone H3 lysine 4 methylation marks (Bernstein et al., 2006). Furthermore, master regulators of pluripotency, like Oct4, Sox2 and Nanog, control genes that encode transcription factors, chromatin-modifying enzymes, and signal-transduction proteins that regulate ES-cell self-renewal (Boyer et al., 2006a; Loh et al., 2006). During the reprogramming process, one or more reprogrammer(s), as specific β-catenin target(s), might initiate the modifications of the chromatin state of the somatic nucleus, finally resulting in the establishment of pluripotent, ES-like genomic features, containing PcG-regulated bivalent domain modifications and Oct4-, Sox2- and Nanog-regulated genes.

In culture, the generation of induced pluripotent stem cells (iPS) demonstrates that pluripotency can be obtained by over-expression of defined transcription factors (Aasen et al., 2008; Aoi et al., 2008; Brambrink et al., 2008; Dimos et al., 2008; Hanna et al., 2008; Hockemeyer et al., 2008; Huangfu et al., 2008b; Kim et al., 2008; Lowry et al., 2008; Maherali et al., 2008; Maherali et al., 2007; Park et al., 2008a; Park et al., 2008b; Stadtfeld et al., 2008a; Stadtfeld et al., 2008b; Takahashi et al., 2007; Takahashi and Yamanaka, 2006; Wernig et al., 2008; Yu et al., 2007), including stem-cell genes; however, this process is not likely to occur *in vivo* since factors like Nanog, Oct4 and Sox2 are not expressed in adult tissues or in adult somatic stem cells, and since they are dispensable for adult stem-cell function (Lengner et al., 2007). On the other hand, over-expression of a blend of these transcription factors is a potent recipe for the generation of novel stem cells in culture that will hopefully be a powerful resource for medical applications. Our data demonstrate that transient and periodic activation of the Wnt/β-catenin signalling pathway without over-expression of any of the known ES-cell genes can control the reprogramming of somatic cells, mimicking a physiological scenario that might occur *in vivo.* Remarkably, adult mouse liver cells express high levels of β-catenin, and the efficiency of selection of iPS-hepatocytes appears to be greater with respect to iPS-fibroblasts, even if the numbers of retroviral integrations of the reprogramming factors is lower in liver cells (Aoi et al., 2008). These findings indicate that Wnt signalling can enhance the generation of iPS-cells. This was recently demonstrated by Marson et al. (2008), where they showed that iPS lines can be isolated with high efficiency from MEFs cells transduced with lentiviruses encoding Oct4, Sox2 and Klf4 and cultured in Wnt3a-CM (Wnt3a-conditioned medium). Of note, and in agreement with our data, Marson et al. (2008) showed that the Wnt-mediated mechanism of reprogramming is independent of c-Myc. However, they were not able to reproduce the effects of Wnt3a-CM on reprogramming with GSK-3 inhibitors. This was potentially due to the prolonged culture in medium containing the inhibitors that, as the authors have shown here, have negative effects on reprogramming.

Isolated reprogrammed clones can differentiate *in vivo* and *in vitro.* The process of achieving full reprogramming is slow, as was seen in iPS cells that expressed detectable amounts of GFP weeks after antibiotic selection (Jaenisch and Young, 2008). Interestingly, in the present study, the selected hybrids were soon fully reprogrammed while they were drug selected, since they expressed high levels of GFP and puromycin at the same time.

One question arises finally: can the Wnt-signalling-mediated reprogramming of somatic cells occur upon spontaneous cell fusion? PEG-independent fusion has been shown for different cell types, but it is a very rare event (Ogle et al., 2005). This is probably because the majority of hybrids formed are forced to undergo differentiation under the culturing conditions used. It will be of interest in the future to determine if reprogramming of spontaneously fused cells can be enhanced by the activation of the Wnt/β-catenin signalling pathway. This might represent the starting point to explore whether the reprogramming of spontaneously fused cells can occur in living organisms.

### References

Aasen, T., Raya, A., Barrero, M. J., Garreta, E., Consiglio, A., Gonzalez, F., Vassena, R., Bilic, J., Pekarik, V., Tiscornia, G., et al. (2008). Efficient and rapid generation of induced pluripotent stem cells from human keratinocytes. Nat Biotechnol 26, 1276-1284.
Anton, R., Kestler, H. A., and Kuhl, M. (2007). beta-Catenin signaling contributes to sternness and regulates early differentiation in murine embryonic stem cells. FEBS Lett 581, 5247-5254.
Aoi, T., Yae, K., Nakagawa, M., Ichisaka, T., Okita, K., Takahashi, K., Chiba, T., and Yamanaka, S. (2008). Generation of Pluripotent Stem Cells from Adult Mouse Liver and Stomach Cells. Science.
Bazzi, H., Fantauzzo, K. A., Richardson, G. D., Jahoda, C. A., and Christiano, A. M. (2007). The Wnt inhibitor, Dickkopf 4, is induced by canonical Wnt signaling during ectodermal appendage morphogenesis. Dev Biol 305, 498-507.
Behrens, J. (2005). The role of the Wnt signalling pathway in colorectal tumorigenesis. Biochem Soc Trans 33, 672-675.
Bernstein, B. E., Mikkelsen, T. S., Xie, X., Kamal, M., Huebert, D. J., Cuff, J., Fry, B., Meissner, A., Wernig, M., Plath, K., et al. (2006). A bivalent chromatin structure marks key developmental genes in embryonic stem cells. Cell 125, 315-326.
Boheler, K. R., Czyz, J., Tweedie, D., Yang, H. T., Anisimov, S. V., and Wobus, A. M. (2002). Differentiation of pluripotent embryonic stem cells into cardiomyocytes. Circ Res 91, 189-201.
Boyer, L. A., Mathur, D., and Jaenisch, R. (2006a). Molecular control of pluripotency. Curr Opin Genet Dev 16, 455-462.
Boyer, L. A., Plath, K., Zeitlinger, J., Brambrink, T., Medeiros, L. A., Lee, T. I., Levine, S. S., Wernig, M., Tajonar, A., Ray, M. K., et al. (2006b). Polycomb complexes repress developmental regulators in murine embryonic stem cells. Nature 441, 349-353.
Brambrink, T., Foreman, R., Welstead, G. G., Lengner, C. J., Wernig, M., Suh, H., and Jaenisch, R. (2008). Sequential expression of pluripotency markers during direct reprogramming of mouse somatic cells. Cell Stem Cell 2, 151-159.
Chia, I. V., and Costantini, F. (2005). Mouse axin and axin2/conductin proteins are functionally equivalent in vivo. Mol Cell Biol 25, 4371-4376.
Cole, M. F., Johnstone, S. E., Newman, J. J., Kagey, M. H., and Young, R. A. (2008). Tcf3 is an integral component of the core regulatory circuitry of embryonic stem cells. Genes Dev 22, 746-755.
Conti, L., Pollard, S. M., Gorba, T., Reitano, E., Toselli, M., Biella, G., Sun, Y., Sanzone, S., Ying, Q. L., Cattaneo, E., and Smith, A. (2005). Niche-independent symmetrical self-renewal of a mammalian tissue stem cell. PLoS Biol 3, e283.
Cowan, C. A., Atienza, J., Melton, D. A., and Eggan, K. (2005). Nuclear reprogramming of somatic cells after fusion with human embryonic stem cells. Science 309, 1369-1373.
Dimos, J. T., Rodolfa, K. T., Niakan, K. K., Weisenthal, L. M., Mitsumoto, H., Chung, W., Croft, G. F., Saphier, G., Leibel, R., Goland, R., et al. (2008). Induced pluripotent stem cells generated from patients with ALS can be differentiated into motor neurons. Science 321, 1218-1221.
Doble, B. W., Patel, S., Wood, G. A., Kockeritz, L. K., and Woodgett, J. R. (2007). Functional redundancy of GSK-3alpha and GSK-3beta in Wnt/beta-catenin signaling shown by using an allelic series of embryonic stem cell lines. Dev Cell 12, 957-971.
Dravid, G., Ye, Z., Hammond, H., Chen, G., Pyle, A., Donovan, P., Yu, X., and Cheng, L. (2005). Defining the role of Wnt/beta-catenin signaling in the survival, proliferation, and self-renewal of human embryonic stem cells. Stem Cells 23, 1489-1501.
Gurley, K. A., Rink, J. C., and Sanchez Alvarado, A. (2008). Beta-catenin defines head versus tail identity during planarian regeneration and homeostasis. Science 319, 323-327.
Haegel, H., Larue, L., Ohsugi, M., Fedorov, L., Herrenknecht, K., and Kemler, R. (1995). Lack of beta-catenin affects mouse development at gastrulation. Development 121, 3529-3537.
Hanna, J., Markoulaki, S., Schorderet, P., Carey, B. W., Beard, C., Wernig, M., Creyghton, M. P., Steine, E. J., Cassady, J. P., Foreman, R., et al. (2008). Direct reprogramming of terminally differentiated mature B lymphocytes to pluripotency. Cell 133, 250-264.
He, T. C., Sparks, A. B., Rago, C., Hermeking, H., Zawel, L., da Costa, L. T., Morin, P. J., Vogelstein, B., and Kinzler, K. W. (1998). Identification of c-MYC as a target of the APC pathway. Science 281, 1509-1512.
Hockemeyer, D., Soldner, F., Cook, E. G., Gao, Q., Mitalipova, M., and Jaenisch, R. (2008). A drug-inducible system for direct reprogramming of human somatic cells to pluripotency. Cell Stem Cell 3, 346-353
Hoppler, S., and Kavanagh, C. L. (2007). Wnt signalling: variety at the core. J Cell Sci 120, 385-393.
Huangfu, D., Maehr, R., Guo, W., Eijkelenboom, A., Snitow, M., Chen, A. E., and Melton, D. A. (2008a). Induction of pluripotent stem cells by defined factors is greatly improved by small-molecule compounds. Nat Biotechnol 26, 795-797.
Huangfu, D., Osafune, K., Maehr, R., Guo, W., Eijkelenboom, A., Chen, S., Muhlestein, W., and Melton, D. A. (2008b). Induction of pluripotent stem cells from primary human fibroblasts with only Oct4 and Sox2. Nat Biotechnol 26, 1269-1275
Huelsken, J., Vogel, R., Brinkmann, V., Erdmann, B., Birchmeier, C., and Birchmeier, W. (2000). Requirement for beta-catenin in anterior-posterior axis formation in mice. J Cell Biol 148, 567-578.
Ito, M., Yang, Z., Andl, T., Cui, C., Kim, N., Millar, S. E., and Cotsarelis, G. (2007). Wnt-dependent de novo hair follicle regeneration in adult mouse skin after wounding. Nature 447, 316-320.
Jaenisch, R., and Young, R. (2008). Stem cells, the molecular circuitry of pluripotency and nuclear reprogramming. Cell 132, 567-582.
Jho, E. H., Zhang, T., Domon, C., Joo, C. K., Freund, J. N., and Costantini, F. (2002). Wnt/beta-catenin/Tcf signaling induces the transcription of Axin2, a negative regulator of the signaling pathway. Mol Cell Biol 22, 1172-1183.
Karner, C., Wharton, K. A., and Carroll, T. J. (2006). Apical-basal polarity, Wnt signaling and vertebrate organogenesis. Semin Cell Dev Biol 17, 214-222.
Katoh, M., and Katoh, M. (2007). WNT signaling pathway and stem cell signaling network. Clin Cancer Res 13, 4042-4045.
Kawano, Y., and Kypta, R. (2003). Secreted antagonists of the Wnt signalling pathway. J Cell Sci 116, 2627-2634.
Kielman, M. F., Rindapaa, M., Gaspar, C., van Poppel, N., Breukel, C., van Leeuwen, S., Taketo, M. M., Roberts, S., Smits, R., and Fodde, R. (2002). Apc modulates embryonic stem-cell differentiation by controlling the dosage of beta-catenin signaling. Nat Genet 32, 594-605.
Kikuchi, A., Yamamoto, H., and Kishida, S. (2007). Multiplicity of the interactions of Wnt proteins and their receptors. Cell Signal 19, 659-671.
Kim, J. B., Zaehres, H., Wu, G., Gentile, L., Ko, K., Sebastiano, V., Arauzo-Bravo, M. J., Ruau, D., Han, D. W., Zenke, M., and Scholer, H. R. (2008). Pluripotent stem cells induced from adult neural stem cells by reprogramming with two factors. Nature.
Lee, T. I., Jenner, R. G., Boyer, L. A., Guenther, M. G., Levine, S. S., Kumar, R. M., Chevalier, B., Johnstone, S. E., Cole, M. F., Isono, K., et al. (2006). Control of developmental regulators by Polycomb in human embryonic stem cells. Cell 125, 301-313.
Lengner, C. J., Camargo, F. D., Hochedlinger, K., Welstead, G. G., Zaidi, S., Gokhale, S., Scholer, H. R., Tomilin, A., and Jaenisch, R. (2007). Oct4 expression is not required for mouse somatic stem cell self-renewal. Cell Stem Cell 1, 403-415.
Logan, C. Y., and Nusse, R. (2004). The Wnt signaling pathway in development and disease. Annu Rev Cell Dev Biol 20, 781-810.
Loh, Y. H., Wu, Q., Chew, J. L., Vega, V. B., Zhang, W., Chen, X., Bourque, G., George, J., Leong, B., Liu, J., et al. (2006). The Oct4 and Nanog transcription network regulates pluripotency in mouse embryonic stem cells. Nat Genet 38, 431-440.
Lowry, W. E., Richter, L., Yachechko, R., Pyle, A. D., Tchieu, J., Sridharan, R., Clark, A. T., and Plath, K. (2008). Generation of human induced pluripotent stem cells from dermal fibroblasts. Proc Natl Acad Sci U S A 105, 2883-2888.
Lustig, B., Jerchow, B., Sachs, M., Weiler, S., Pietsch, T., Karsten, U., van de Wetering, M., Clevers, H., Schlag, P. M., Birchmeier, W., and Behrens, J. (2002). Negative feedback loop of Wnt signaling through upregulation of conductin/axin2 in colorectal and liver tumors. Mol Cell Biol 22, 1184-1193.
Maherali, N., Sridharan, R., Xie, W., Utikal, J., Eminli, S., Arnold, K., Stadtfeld, M., Yachechko, R., Tchieu, J., Jaenisch, R., et al. (2007). Directly reprogrammed fibroblasts show global epigenetic remodeling and widespread tissue contribution. Cell Stem Cell 1, 55-70.
Maherali, N., Ahfeldt, T., Rigamonti, A., Utikal, J., Cowan, C., and Hochedlinger, K. (2008). A high-efficiency system for the generation and study of human induced pluripotent stem cells. Cell Stem Cell 3, 340-345.
Marson, A., Foreman, R., Chevalier, B., Bilodeau, S., Kahn, M., Young, R. A., and Jaenisch, R. (2008). Wnt signaling promotes reprogramming of somatic cells to pluripotency. Cell Stem Cell 3, 132-135.
Meijer, L., Skaltsounis, A. L., Magiatis, P., Polychronopoulos, P., Knockaert, M., Leost, M., Ryan, X. P., Vonica, C. A., Brivanlou, A., Dajani, R., et al. (2003). GSK-3-selective inhibitors derived from Tyrian purple indirubins. Chem Biol 10, 1255-1266.
Niida, A., Hiroko, T., Kasai, M., Furukawa, Y., Nakamura, Y., Suzuki, Y., Sugano, S., and Akiyama, T. (2004). DKK1, a negative regulator of Wnt signaling, is a target of the beta-catenin/TCF pathway. Oncogene 23, 8520-8526.
Ogle, B. M., Cascalho, M., and Platt, J. L. (2005). Biological implications of cell fusion. Nat Rev Mol Cell Biol 6, 567-575.
Osakada, F., Ooto, S., Akagi, T., Mandai, M., Akaike, A., and Takahashi, M. (2007). Wnt signaling promotes regeneration in the retina of adult mammals. J Neurosci 27, 4210-4219. Park, I. H., Zhao, R., West, J. A., Yabuuchi, A., Huo, H., Ince, T. A., Lerou, P. H., Lensch, M. W., and Daley, G. Q. (2008). Reprogramming of human somatic cells to pluripotency with defined factors. Nature 451, 141-146.
Petersen, C. P., and Reddien, P. W. (2008). Smed-betacatenin-1 is required for anteroposterior blastema polarity in planarian regeneration. Science 319, 327-330.
Reya, T., and Clevers, H. (2005). Wnt signalling in stem cells and cancer. Nature 434, 843-850.
Reya, T., Duncan, A. W., Ailles, L., Domen, J., Scherer, D. C., Willert, K., Hintz, L., Nusse, R., and Weissman, I. L. (2003). A role for Wnt signalling in self-renewal of haematopoietic stem cells. Nature 423, 409-414.
Sato, N., Meijer, L., Skaltsounis, L., Greengard, P., and Brivanlou, A. H. (2004). Maintenance of pluripotency in human and mouse embryonic stem cells through activation of Wnt signaling by a pharmacological GSK-3-specific inhibitor. Nat Med 10, 55-63.
Silva, J., Chambers, I., Pollard, S., and Smith, A. (2006). Nanog promotes transfer of pluripotency after cell fusion. Nature 441, 997-1001.
Stadtfeld, M., Brennand, K., and Hochedlinger, K. (2008a). Reprogramming of pancreatic beta cells into induced pluripotent stem cells. Curr Biol 18, 890-894.
Stoick-Cooper, C. L., Moon, R. T., and Weidinger, G. (2007). Advances in signaling in vertebrate regeneration as a prelude to regenerative medicine. Genes Dev 21, 1292-1315.
Tada, M., Tada, T., Lefebvre, L., Barton, S. C., and Surani, M. A. (1997). Embryonic germ cells induce epigenetic reprogramming of somatic nucleus in hybrid cells. Embo J 16, 6510-6520.
Tada, M., Takahama, Y., Abe, K., Nakatsuji, N., and Tada, T. (2001). Nuclear reprogramming of somatic cells by in vitro hybridization with ES cells. Curr Biol 11, 1553-1558.
Takahashi, K., Tanabe, K., Ohnuki, M., Narita, M., Ichisaka, T., Tomoda, K., and Yamanaka, S. (2007). Induction of Pluripotent Stem Cells from Adult Human Fibroblasts by Defined Factors. Cell.
Takahashi, K., and Yamanaka, S. (2006). Induction of pluripotent stem cells from mouse embryonic and adult fibroblast cultures by defined factors. Cell 126, 663-676.
Ueno, S., Weidinger, G., Osugi, T., Kohn, A. D., Golob, J. L., Pabon, L., Reinecke, H., Moon, R. T., and Murry, C. E. (2007). Biphasic role for Wnt/beta-catenin signaling in cardiac specification in zebrafish and embryonic stem cells. Proc Natl Acad Sci U S A 104, 9685-9690.
Vajta, G. (2007). Somatic cell nuclear transfer in its first and second decades: successes, setbacks, paradoxes and perspectives. Reprod Biomed Online 15, 582-590.
Wernig, M., Lengner, C. J., Hanna, J., Lodato, M. A., Steine, E., Foreman, R., Staerk, J., Markoulaki, S., and Jaenisch, R. (2008). A drug-inducible transgenic system for direct reprogramming of multiple somatic cell types. Nat Biotechnol 26, 916-924.
Wharton, K. A., Jr., Zimmermann, G., Rousset, R., and Scott, M. P. (2001). Vertebrate proteins related to Drosophila Naked Cuticle bind Dishevelled and antagonize Wnt signaling. Dev Biol 234, 93-106.
Wiese, C., Rolletschek, A., Kania, G., Navarrete-Santos, A., Anisimov, S. V., Steinfarz, B., Tarasov, K. V., Brugh, S. A., Zahanich, I., Ruschenschmidt, C., et al. (2006). Signals from embryonic fibroblasts induce adult intestinal epithelial cells to form nestin-positive cells with proliferation and multilineage differentiation capacity in vitro. Stem Cells 24, 2085-2097. Willert, K., and Jones, K. A. (2006). Wnt signaling: is the party in the nucleus? Genes Dev 20, 1394-1404.
Yan, D., Wiesmann, M., Rohan, M., Chan, V., Jefferson, A. B., Guo, L., Sakamoto, D., Caothien, R. H., Fuller, J. H., Reinhard, C., et al. (2001). Elevated expression of axin2 and hnkd mRNA provides evidence that Wnt/beta -catenin signaling is activated in human colon tumors. Proc Natl Acad Sci U S A 98, 14973-14978.
Yeom, Y. I., Fuhrmann, G., Ovitt, C. E., Brehm, A., Ohbo, K., Gross, M., Hubner, K., and Scholer, H. R. (1996). Germline regulatory element of Oct-4 specific for the totipotent cycle of embryonal cells. Development 122, 881-894.
Yokoyama, H., Ogino, H., Stoick-Cooper, C. L., Grainger, R. M., and Moon, R. T. (2007). Wnt/beta-catenin signaling has an essential role in the initiation of limb regeneration. Dev Biol 306, 170-178.
Yu, J., Vodyanik, M. A., Smuga-Otto, K., Antosiewicz-Bourget, J., Frane, J. L., Tian, S., Nie, J., Jonsdottir, G. A., Ruotti, V., Stewart, R., et al. (2007). Induced Pluripotent Stem Cell Lines Derived from Human Somatic Cells. Science.
Zito, E., Fraldi, A., Pepe, S., Annunziata, I., Kobinger, G., Di Natale, P., Ballabio, A., and Cosma, M. P. (2005). Sulphatase activities are regulated by the interaction of sulphatase-modifying factor 1 with SUMF2. EMBO Rep 6, 655-660.
Zito, E., Buono, M., Pepe, S., Settembre, C., Annunziata, I., Surace, E. M., Dierks, T., Monti, M., Cozzolino, M., Pucci, P., et al. (2007). Sulfatase modifying factor 1 trafficking through the cells: from endoplasmic reticulum to the endoplasmic reticulum. Embo J 26, 2443-2453.

## Claims

1. An in vitro method for reprogramming an isolated differentiated cell to an undifferentiated stem cell comprising the step of fusing said differentiated cell to an isolated pluripotent cell, wherein said isolated pluripotent cell is treated in vitro with a suitable amount of a Wnt/β-catenin pathway activator being at least one Wnt protein isoform or a glycogen synthase kinase-3 inhibitor.

2. The method according to claim 1 wherein the isolated pluripotent cell is treated in vitro before or after the step of fusing said differentiated cell to said pluripotent cell.

3. The method according to any of previous claims wherein the differentiated cell is selected from the group of: somatic cell, neural stem cell, embryonic fibroblast, thymocyte.

4. The method according to any of previous claims wherein the isolated pluripotent cell is selected from the group of: adult stem cell, embryonic stem cell.

5. The method according to any of previous claims wherein the isolated pluripotent cell is a mouse or a human cell and the differentiated cell is a mouse or a human cell.

6. The method according to any of previous claims wherein the Wnt protein isoform is selected from the group of: Wnt1, Wnt2, Wnt2b/13, Wnt3, Wnt3a, Wnt4, Wnt5a, Wnt5b, Wnt6, Wnt7a, Wnt8a, Wnt8b, Wnt9a, Wnt9b, Wnt10a, Wnt10b, Wnt11 or Wnt16.

7. The method according to any of previous claims wherein the glycogen synthase kinase-3 inhibitor is BIO or CHIR99021 or an analogue thereof.

8. The method according to any of previous claims further comprising overexpressing Nanog protein in the isolated pluripotent cell.

9. The method according to claims 1 to 7 further comprising overexpressing Nanog protein in the fused cell.

10. The method according to claim 8 or 9 wherein the overexpression of Nanog protein is obtained by genetically modifying the isolated pluripotent cell or the fused cell or by transducing the isolated pluripotent cell or the fused cell with an appropriated vector.

## Patentansprüche

1. In-vitro Verfahren zum Reprogrammieren einer isolierten differenzierten Zelle in eine undifferenzierte Stammzelle, das den Schritt des Verschmelzens der differenzierten Zelle mit einer isolierten pluripotenten Zelle umfasst, wobei die isolierte pluripotente Zelle in vitro mit einer geeigneten Menge eines Aktivators des Wnt/β-Catenin-Wegs behandelt wird, welcher mindestens ein Wnt Proteinisoform oder ein Glycogensynthasekinase-3 Inhibitor ist.

2. Verfahren nach Anspruch 1, wobei die isolierte pluripotente Zelle in vitro behandelt wird vor oder nach dem Schritt des Verschmelzens der differenzierten Zelle mit der pluripotenten Zelle.

3. Verfahren nach einem der vorherigen Ansprüche, wobei die differenzierte Zelle ausgewählt wird aus der Gruppe einer(s): somatischen Zelle, neuralen Stammzelle, embryonalen Fibroblasten, Thymocyte.

4. Verfahren nach einem der vorherigen Ansprüche, wobei die isolierte pluripotente Zelle ausgewählt wird aus der Gruppe einer: adulten Stammzelle, embryonalen Stammzelle.

5. Verfahren nach einem der vorherigen Ansprüche, wobei die isolierte pluripotente Zelle eine Mäuse- oder eine menschliche Zelle und die differenzierte Zelle eine Mäuse- oder eine menschliche Zelle ist.

6. Verfahren nach einem der vorherigen Ansprüche, wobei das Wnt-Proteinisoform ausgewählt wird aus der Gruppe von: Wnt1, Wnt2, Wnt2b/13, Wnt3, Wnt3a, Wnt4, Wnt5a, Wnt5b, Wnt6, Wnt7a, Wnt8a, Wnt8b, Wnt9a, Wnt9b, Wnt10a, Wnt10b, Wnt11 oder Wnt16.

7. Verfahren nach einem der vorherigen Ansprüche, wobei der Glycogensynthasekinase-3 Inhibitor BIO oder CHIR99021 oder ein Analog davon ist.

8. Verfahren nach einem der vorherigen Ansprüche, das ferner das Überexprimieren des Nanog Proteins in der isolierten pluripotenten Zelle umfasst.

9. Verfahren nach einem der Ansprüche 1 bis 7, das ferner das Überexprimieren des Nanog Proteins in der verschmolzenen Zelle umfasst.

10. Verfahren nach Anspruch 8 oder 9, wobei die Überexpression des Nanog Proteins erhalten wird durch genetisches Modifizieren der isolierten pluripotenten Zelle oder der verschmolzenen Zelle oder durch Transduzieren der isolierten pluripotenten Zelle oder der verschmolzenen Zelle mit einem bereitgestellten Vektor.

## Revendications

1. Procédé *in vitro* destiné à la reprogrammation d'une cellule différenciée isolée en une cellule souche indifférenciée, comprenant l'étape de fusion de ladite cellule différenciée avec une cellule pluripotente isolée, où ladite cellule pluripotente isolée est traitée *in vitro* avec une quantité appropriée d'un activateur de la voie Wnt/β-caténine étant au moins une isoforme de la protéine Wnt ou un inhibiteur de la glycogène synthase kinase 3.

2. Procédé selon la revendication 1, dans lequel la cellule pluripotente isolée est traitée *in vitro* avant ou après l'étape de fusion de ladite cellule différenciée avec ladite cellule pluripotente.

3. Procédé selon l'une quelconque des revendications précédentes, dans lequel la cellule différenciée est sélectionnée dans le groupe comprenant les cellules somatiques, les cellules souches neuronales, les fibroblastes embryonnaires et les thymocytes.

4. Procédé selon l'une quelconque des revendications précédentes, dans lequel la cellule pluripotente isolée est sélectionnée dans le groupe comprenant les cellules souches adultes, les cellules souches embryonnaires.

5. Procédé selon l'une quelconque des revendications précédentes, dans lequel la cellule pluripotente isolée est une cellule de souris ou une cellule humaine et la cellule différenciée est une cellule de souris ou une cellule humaine.

6. Procédé selon l'une quelconque des revendications précédentes, dans lequel l'isoforme de la protéine Wnt est sélectionnée dans le groupe comprenant Wnt1, Wnt2, Wnt2b/13, Wnt3, Wnt3a, Wnt4, Wnt5a, Wnt5b, Wnt6, Wnt7a, Wnt8a, Wnt8b, Wnt9a, Wnt9b, Wnt10a, Wnt10b, Wnt11 ou Wnt16.

7. Procédé selon l'une quelconque des revendications précédentes, dans lequel l'inhibiteur de la glycogène synthase kinase 3 est BIO ou CHIR99021 ou un analogue de ceux-ci.

8. Procédé selon l'une quelconque des revendications précédentes comprenant en outre une surexpression de la protéine Nanog dans la cellule pluripotente isolée.

9. Procédé selon l'une quelconque des revendications 1 à 7 comprenant en outre une surexpression de la protéine Nanog dans la cellule fusionnée.

10. Procédé selon la revendication 8 ou 9, dans lequel la surexpression de la protéine Nanog est obtenue en modifiant génétiquement la cellule pluripotente isolée ou la cellule fusionnée ou en effectuant une transduction de la cellule pluripotente isolée ou de la cellule fusionnée à l'aide d'un vecteur approprié.


### SEQUENCE LISTING

<110> Fondazione Telethon
   Cosma, Maria Pia
   Lluis Vinas, Frederic
<120> Method for reprogramming differentiated cells
<130> BE 101669
<160> 44
<170> PatentIn version 3.5
<210> 1
   <211> 24
   <212> DNA
   <213> Artificial Sequence
<220>
   <223> Synthetic primer
<400> 1
   ggcgttctct ttggaaaggt gttc 24
<210> 2
   <211> 20
   <212> DNA
   <213> Artificial Sequence
<220>
   <223> Synthetic primer
<400> 2
   ctcgaaccac atccttctct 20
<210> 3
   <211> 24
   <212> DNA
   <213> Artificial Sequence
<220>
   <223> Synthetic primer
<400> 3
   agggtctgct actgagatgc tctg 24
<210> 4
   <211> 24
   <212> DNA
   <213> Artificial Sequence
<220>
   <223> Synthetic primer
<400> 4
   caaccactgg tttttctgcc accg 24
<210> 5
   <211> 22
   <212> DNA
   <213> Artificial Sequence
<220>
   <223> Synthetic primer
<400> 5
   gccctcgaca gactgaccct aa 22
<210> 6
   <211> 22
   <212> DNA
   <213> Artificial Sequence
<220>
   <223> Synthetic primer
<400> 6
   cttcctcagg gcggttttac cc 22
<210> 7
   <211> 20
   <212> DNA
   <213> Artificial Sequence
<220>
   <223> Synthetic primer
<400> 7
   gactacctgc tgggcctcaa 20
<210> 8
   <211> 20
   <212> DNA
   <213> Artificial Sequence
<220>
   <223> Synthetic primer
<400> 8
   cgacactcgg ttccccttct 20
<210> 9
   <211> 20
   <212> DNA
   <213> Artificial Sequence
<220>
   <223> Synthetic primer
<400> 9
   gcgtgggtat cagaagcact 20
<210> 10
   <211> 20
   <212> DNA
   <213> Artificial Sequence
<220>
   <223> Synthetic primer
<400> 10
   ccagtcgggt aagaaaccaa 20
<210> 11
   <211> 20
   <212> DNA
   <213> Artificial Sequence
<220>
   <223> Synthetic primer
<400> 11
   gggtaagacc cgagttcctc 20
<210> 12
   <211> 20
   <212> DNA
   <213> Artificial Sequence
<220>
   <223> Synthetic primer
<400> 12
   atcaccactt tgccaccttc 20
<210> 13
   <211> 21
   <212> DNA
   <213> Artificial Sequence
<220>
   <223> Synthetic primer
<400> 13
   gggagcagtt ttgtggcagc a 21
<210> 14
   <211> 23
   <212> DNA
   <213> Artificial Sequence
<220>
   <223> Synthetic primer
<400> 14
   agggtcctgg gtaaatgggt gag 23
<210> 15
   <211> 21
   <212> DNA
   <213> Artificial Sequence
<220>
   <223> Synthetic primer
<400> 15
   tgccgcccac tctccccaac c 21
<210> 16
   <211> 21
   <212> DNA
   <213> Artificial Sequence
<220>
   <223> Synthetic primer
<400> 16
   ccgccgccgt catcgtcttc c 21
<210> 17
   <211> 18
   <212> DNA
   <213> Artificial Sequence
<220>
   <223> Synthetic primer
<400> 17
   tgctgcctgt gagtcata 18
<210> 18
   <211> 20
   <212> DNA
   <213> Artificial Sequence
<220>
   <223> Synthetic primer
<400> 18
   acaagaggct gtagaacatg 20
<210> 19
   <211> 19
   <212> DNA
   <213> Artificial Sequence
<220>
   <223> Synthetic primer
<400> 19
   gctctcctgc tttcccagc 19
<210> 20
   <211> 26
   <212> DNA
   <213> Artificial Sequence
<220>
   <223> Synthetic primer
<400> 20
   ctcccatccc tactgccttc tgcagc 26
<210> 21
   <211> 18
   <212> DNA
   <213> Artificial Sequence
<220>
   <223> Synthetic primer
<400> 21
   tccctcatcc tcctgcta 18
<210> 22
   <211> 19
   <212> DNA
   <213> Artificial Sequence
<220>
   <223> Synthetic primer
<400> 22
   gcacattctt ctccgtcac 19
<210> 23
   <211> 20
   <212> DNA
   <213> Artificial Sequence
<220>
   <223> Synthetic primer
<400> 23
   tctacacaga ccaccaacgc 20
<210> 24
   <211> 20
   <212> DNA
   <213> Artificial Sequence
<220>
   <223> Synthetic primer
<400> 24
   agaaactcct ccttgacggg 20
<210> 25
   <211> 21
   <212> DNA
   <213> Artificial Sequence
<220>
   <223> Synthetic primer
<400> 25
   aaagtcaatg gctcccacga a 21
<210> 26
   <211> 21
   <212> DNA
   <213> Artificial Sequence
<220>
   <223> Synthetic primer
<400> 26
   cttcagtctg tacttcactg g 21
<210> 27
   <211> 20
   <212> DNA
   <213> Artificial Sequence
<220>
   <223> Synthetic primer
<400> 27
   actcccactc ttccaccttc 20
<210> 28
   <211> 19
   <212> DNA
   <213> Artificial Sequence
<220>
   <223> Synthetic primer
<400> 28
   tcttgctcag tgtccttgc 19
<210> 29
   <211> 30
   <212> DNA
   <213> Artificial Sequence
<220>
   <223> Synthetic primer
<400> 29
   gattttgtag gtgggattaa ttgtgaattt 30
<210> 30
   <211> 30
   <212> DNA
   <213> Artificial Sequence
<220>
   <223> Synthetic primer
<400> 30
   accaaaaaaa cccacactca tatcaatata 30
<210> 31
   <211> 24
   <212> DNA
   <213> Artificial Sequence
<220>
   <223> Synthetic primer
<400> 31
   ggttttttag aggatggttg agtg 24
<210> 32
   <211> 23
   <212> DNA
   <213> Artificial Sequence
<220>
   <223> Synthetic primer
<400> 32
   tccaacccta ctaacccatc acc 23
<210> 33
   <211> 20
   <212> DNA
   <213> Artificial Sequence
<220>
   <223> Synthetic primer
<400> 33
   ctgcgagagt tcccagaaga 20
<210> 34
   <211> 20
   <212> DNA
   <213> Artificial Sequence
<220>
   <223> Synthetic primer
<400> 34
   ttcctgttct ccagctcaca 20
<210> 35
   <211> 21
   <212> DNA
   <213> Artificial Sequence
<220>
   <223> Synthetic primer
<400> 35
   gctcagtcat cagccaactc g 21
<210> 36
   <211> 20
   <212> DNA
   <213> Artificial Sequence
<220>
   <223> Synthetic primer
<400> 36
   atcacaggcg aagtccaatc 20
<210> 37
   <211> 20
   <212> DNA
   <213> Artificial Sequence
<220>
   <223> synthetic primer
<400> 37
   gcagacggga gtttctcctc 20
<210> 38
   <211> 20
   <212> DNA
   <213> Artificial Sequence
<220>
   <223> synthetic primer
<400> 38
   tcaagcatac ctcgggtttc 20
<210> 39
   <211> 20
   <212> DNA
   <213> Artificial Sequence
<220>
   <223> synthetic primer
<400> 39
   cgactaagtt ggagggaacg 20
<210> 40
   <211> 20
   <212> DNA
   <213> Artificial Sequence
<220>
   <223> synthetic primer
<400> 40
   ctttgtccac gtggtcctct 20
<210> 41
   <211> 19
   <212> DNA
   <213> Artificial Sequence
<220>
   <223> synthetic primer
<400> 41
   gtcgtaatgc cgagggtga 19
<210> 42
   <211> 19
   <212> DNA
   <213> Artificial Sequence
<220>
   <223> synthetic primer
<400> 42
   tccttccgca ttgcaagag 19
<210> 43
   <211> 24
   <212> DNA
   <213> Artificial Sequence
<220>
   <223> synthetic primer
<400> 43
   aaccaaagga tgaagtgcaa gcgg 24
<210> 44
   <211> 24
   <212> DNA
   <213> Artificial Sequence
<220>
   <223> synthetic primer
<400> 44
   tccaagttgg gttggtccaa gtct 24